# EUROPEAN PATENT SPECIFICATION

(11) **EP 1 269 194 B1**
(45) Date of publication and mention of the grant of the patent: **07.03.2007**
(21) Application number: 01922478.1
(22) Date of filing: 19.03.2001
(51) Int. Cl.: G01N 33/574, G01N 33/68, G06F 19/00, C07K 14/82, C07K 14/47

(54) **PROSTATE CANCER MARKERS**
PROSTATAKREBS-MARKER
MARQUEURS DU CANCER DE LA PROSTATE

(30) Priority: 20.03.2000 US 190725 P
(43) Date of publication of application: 02.01.2003
(73) Proprietor: Eastern Virginia Medical School, Norfolk, VA 23507 (US)
(72) Inventor: WRIGHT, George, L., Jr., Virginia Beach, VA 23455 (US); ADAM, Bao-Ling, Norfolk, VA 23508 (US); CAZARES, Lisa, H., Chesapeake, VA 23322 (US)
(74) Representative: MacLean, Martin Robert
(86) International application number: PCT/US2001/008797
(87) International publication number: WO 2001/071360

(56) References cited:
- WO-A-00/49410
- WO-A-01/25791
- WO-A-99/45398
- DEPERTHES DAVID ET AL: "Isolation of prostatic kallikrein hK2, also known as hGK-1, in human seminal plasma." BIOCHIMICA ET BIOPHYSICA ACTA, vol. 1245, no. 3, 1995, pages 311-316, XP001066231 ISSN: 0006-3002
- WRIGHT G.L. JR. ET AL: "ProteinChip.RTM. surface enhanced laser desorption/ionization ( SELDI ) mass spectrometry: A novel protein biochip technology for detection of prostate cancer biomarkers in complex protein mixtures." PROSTATE CANCER AND PROSTATIC DISEASES, ( 1999 ) 2/5-6 (264-276). , XP008001602
- BEDZYK W.D. ET AL: "Molecular mass determination for prostate -specific antigen and.alpha.1- antichymotrypsin complexed in vitro." BIOTECHNOLOGY AND APPLIED BIOCHEMISTRY, ( 1998 ) 27/3 (249-257). , XP008001618
- PAWELETZ, CLOUD P. ET AL: "Rapid protein display profiling of cancer progression directly from human tissue using a protein biochip" DRUG DEV. RES., 49(1), 34-42 , January 2000 (2000-01), XP002162317
- EMMERT-BUCK M R ET AL: "PROTEIN FINGERPRINTING OF LCM-DISSECTED HUMAN ESOPHAGEAL PROSTATE CANCER BY 2D-PAGE" PROCEEDINGS OF THE ANNUAL MEETING OF THE AMERICAN ASSOCIATION FOR CANCER RESEARCH, NEW YORK, NY, US, vol. 40, no. 526, 1999, page 526 XP000929253 ISSN: 0197-016X
- BANKS R E ET AL: "THE POTENTIAL USE OF LASER CAPTURE MICRODISSECTION TO SELECTIVELY OBTAIN DISTINCT POPULATIONS OF CELLS FOR PROTEOMIC ANALYSIS - PRELIMINARY FINDINGS" ELECTROPHORESIS, WEINHEIM, DE, vol. 20, no. 4/5, April 1999 (1999-04), pages 689-700, XP000925546 ISSN: 0173-0835

## Description

### BACKGROUND OF THE INVENTION

Prostate cancer is the most common form of cancer in males. It typically afflicts aging males, but it can afflict males of all ages. A significant number of males die from prostate cancer every year, and it is the second leading cause of cancer deaths in men. Early diagnosis of prostate cancer in patients reduces the likelihood of death.

The effectiveness of any diagnostic test depends upon its specificity and selectivity. That is, what is the relative ratio of true positive diagnoses, true negative diagnoses, false positive diagnoses and false negative diagnoses? Methods of increasing the percentage of true positive and true negative diagnoses for any condition are desirable medical goals. In the case of prostate cancer, the present diagnostic tests are not completely satisfactory, in that they provide significant numbers of false positive and false negative results.

Conventionally, prostate cancer is diagnosed using prostate specific antigen (PSA) as a marker. In general, PSA levels above 4 ng/ml are suggestive of prostate cancer while levels above 10 ng/ml are highly suggestive of prostate cancer. However, if the cancer is in its early stages, some prostate cancer patients exhibit normal PSA levels at the time of diagnosis. Since conventional PSA tests detect abnormal levels of PSA, conventional PSA tests may not be able to detect the presence of prostate cancer if it is in its early stages. This results in a false negative diagnosis. The inability of conventional PSA tests to diagnose the presence of prostate cancer in some instances (*e.g*., in the early stages of the disease) can be detrimental to the patient. Moreover, many individuals with elevated levels of PSA in the blood serum may not have prostate cancer, but may instead have benign prostate hyperplasia (BPH) (*i.e*., a benign tumor). This results in a false positive diagnosis. In order to determine if a person has prostate cancer, rather than BPH, additional immunoassays using other antibodies and/or biopsies of the prostate tissue are performed. These additional tests are time consuming and expensive for both patients and their care providers.

There is some consensus in the medical community that better diagnosis will result from the discovery of more disease markers that can be used alone or in combination to increase the specificity and selectivity of diagnostic tests.

Wright G.L. *et al.* (Prostate Cancer and Prostatic Diseases, 1992, vol. 2, 264-276) demonstrate the use of the SELDI ProteinChip® for detecting biomarkers of prostatic cancer; and Bedzyk W.D. *et al.* (Biotechnol. Appl. Biochem., 1998, vol. 27, 249-257) provide molecular mass determinations of prostate cancer markers (e.g. PSA) using matrix-assisted laser desorption ionisation mass spectroscopy (MALDI-MS).

WO 99/45398 and WO 01/25791 describe biomarkers of prostate disease that can be used to diagnose patients with prostate cancer and benign prostate hyperplasia from healthy individuals. WO 01/25791 was published after the priority date of the invention claimed herein.

The present invention is defined by independent claims 1 and 38 and by the claims depending on them. The present invention provides a method for aiding in a differential diagnosis of prostate cancer, benign prostate hyperplasia, and a negative diagnosis, comprising:
(a) detecting at least one protein marker in a first sample from a subject, wherein the protein marker is selected from: Marker SP1: 9,402.68 ± 8.97 Da, and Marker SP3: 54,979.27 ± 408.78 Da; and
(b) correlating the detection of the marker or markers from step (a) with a probable diagnosis of prostate cancer, benign prostate hyperplasia or a negative diagnosis, wherein the correlation takes into account the relative detectability of the marker or markers in each diagnosis.

The present invention provides also a kit for aiding in the differential diagnosis of prostate cancer, benign prostate hyperplasia, and a negative diagnosis, comprising:
(1) an adsorbent attached to a substrate, wherein the adsorbent retains at least one protein marker selected from:
   Marker SP1: 9,402.68 ± 8.97 Da, and
   Marker SP3: 54,979.27 ± 408.78 Da; and
(2) instructions to detect the marker or markers by contacting a sample with the adsorbent and detecting the marker or markers retained by the adsorbent wherein said adsorbent comprises at least one antibody selected from an antibody that specifically binds Marker SP1 and an antibody that specifically binds Marker SP3.

### SUMMARY OF THE INVENTION

Several organic biomolecules (*e.g.,* proteins) have been discovered that function as markers in prostate cancer ("CaP") or benign prostate hyperplasia ("BPH") versus a negative diagnosis. Compared to a negative diagnosis, the markers are, variously, more frequently detected, less frequently detected, or differentially detected. The measurement of these markers, alone or in combination, in patient samples provides information that the diagnostician can correlate with probable diagnoses of prostate cancer, benign prostate hyperplasia or with a negative diagnosis (*e.g.,* normal or disease-free). All the markers are characterized by molecular weight They can be resolved from other proteins and other organic biomolecules in a sample by chromatographic separation coupled with, *e.g.,* mass spectrometry. In preferred embodiments, the method of resolution involves Surface-Enhanced Laser Desorption/Ionization mass spectrometry, in which the surface of the mass spectrometry probe plays an active role in the desorption and ionization of the analyte.

A first set of markers was identified in seminal plasma. Marker Set 1 includes the following: Marker SP1: 9,402.68 ± 8.97 Da, pI ~5; Marker SP2: 26,155.30 ± 202.01 Da, pI ~5; Marker SP3:54,979.27 ± 408.78 Da; pI ~9; Marker SP4: 9,752.30 ± 15.08 Da; Marker SP5: 87,66.93 ± 14.44 Da; Marker SP6: 62,77.97 ± 12.36 Da and Marker SP7: 2,781.72 ± 4.41 Da, pI ~9. Compared to a negative diagnosis, SP1 is more frequently detected in CaP, and less frequently detected (*e.g.,* undetected) in BPH. SP2 is more frequently detected in CaP and in BPH. SP3 is more frequently detected in CaP, and less frequently detected in BPH. SP4 is less frequently detected in both CaP and BPH. SP5, SP6 and SP7 are less frequently detected (e.g., undetected) in CaP and less frequently detected in BPH.

A second set of markers was identified in prostate epithelial cell lysates derived from laser capture microdissection. Marker Set 2 includes the following: Marker CL1: 8,494.30 ± 10.24 Da, Marker CL2: 9,614.62 ± 52.19 Da, Marker CL3: 28,472 ± 127.40 Da, and Marker CL4: 33,386.85 ± 160.47 Da. These markers are further characterized by the ability to bind to anion exchange or metal chelate adsorbents washed with an eluant at neutral pH, salt at medium concentration and non-ionic detergent at low concentration. This implies that they are negatively charged at neutral pH. Compared to a negative diagnosis, Marker CL1 and Marker CL2 are more frequently detected in both CaP and BPH. (CL2 is not detected in negative diagnosis.) Markers CL3 and CL4 are detected in all three states. However, a ratio of Markers CL4:CL3 of greater than about 0.4 correlates strongly with CaP.

While the absolute identity of these markers is not know presently, such knowledge is not necessary to measure them in a patient sample because they are sufficiently characterized by mass and by affinity characteristics. We point out that molecular weight and relative pI are characteristic properties of these markers and not limitations on means of detection or isolation. Furthermore, once partial amino acid sequences are obtained, their absolute identify can be determined by matching the sequence in a protein database.

Disclosed is a method for aiding in a diagnosis of prostate cancer or benign prostate hyperplasia. The method involves: a) detecting at least one protein marker selected from Marker Set 1 or Marker Set 2 (as defined herein) in a sample from a subject; and b) correlating the detection of the marker or markers with a probable diagnosis of prostate cancer, benign prostate hyperplasia or a negative diagnosis, wherein the correlation takes into account the relative detectability of the marker or markers in each diagnosis.

In certain embodiments, the sample can be selected from seminal plasma, blood, serum, urine, prostatic fluid, seminal fluid, semen, and prostate tissue. In other embodiments, the marker or markers are detected by gas phase ion spectrometry or, more particularly, laser desorption mass spectrometry. In another embodiment, the marker or markers are detected by immunoassay. In other embodiments, the method comprises detecting a plurality of the markers. In other embodiments, the method involves detecting at least marker SP2 or further detecting at least either or both of SP1 and a third marker selected from SP4, SP5, SP6 and SP7. In another embodiment the method involves detecting marker CL1 and/or marker CL2. In another embodiment the method involves measuring the amount of Marker CL3 and Marker CL4, and determining the ratio of the amounts of Marker CL4 to Marker CL3. In another embodiment the method comprises: i) generating data on the sample with the mass spectrometer indicating intensity of signal for mass/charge ratio, ii) transforming the data into computer-readable form; and iii) executing an algorithm with a programmable digital computer, wherein the algorithm determines closeness-of-fit between the computer-readable data and data indicating a diagnosis of CaP, BPH or a negative diagnosis

Also disclosed is a method for detecting at least one protein marker from Marker Set 1 or Marker Set 2 in a sample, wherein the method comprises detecting the marker or markers by gas phase ion spectrometry.

In one embodiment the method comprises: i) generating data on the sample with a mass spectrometer indicating intensity of signal for mass/charge ratio, ii) transforming the data into computer-readable form; and iii) executing an algorithm with a programmable digital computer wherein the algorithm detects signal in the computer-readable data representing the marker or markers.

In another embodiment the method further comprises, before detecting the markers, fractionating the sample by size exclusion chromatography and collecting a fraction that includes the marker or markers. In another embodiment, the method comprises further comprises, before detecting the markers, fractionating the sample by anion exchange chromatography and collecting a fraction that includes the marker or markers.

In another embodiment the method further comprises i) before detecting the marker or markers, fractionating a sample comprising the marker or markers by contacting the sample with a substrate comprising an adsorbent that retains the marker or markers and removing unretained sample; and ii) desorbing and ionizing the retained markers from the adsorbent during mass spectrometry. In certain embodiments of this method the substrate is a mass spectrometer probe comprising the adsorbent on a probe surface. In another embodiment, the substrate is a resin, and, after fractionating the sample, the resin with the marker or markers retained by the adsorbent is placed on a mass spectrometer probe for desorption and ionization by the mass spectrometer. In another embodiment the adsorbent is selected from a hydrophilic adsorbent, e.g., an anionic adsorbent, and a metal chelate adsorbent, *e.g*., a nickel chelate adsorbent.

In one embodiment involving laser desorption/ionization mass spectrometry the method comprises: i) providing a probe adapted for use with a mass spectrometer comprising an adsorbent attached thereto; ii) contacting the marker or markers with the adsorbent; and iii) desorbing and ionizing the marker or markers from the probe and detecting the desorbed/ionized marker or markers with the mass spectrometer. In another embodiment the method comprises: i) providing a substrate comprising an adsorbent attached thereto; ii) contacting the marker or markers with the adsorbent; iii) placing the substrate on a probe adapted for use with a mass spectrometer comprising an adsorbent attached thereto; and iv) desorbing and ionizing the marker or markers from the probe and detecting the desorbed/ionized marker or markers with the mass spectrometer. In other embodiments the adsorbent is a hydrophilic adsorbent (*e.g*., silicon oxide) or a metal chelate adsorbent (e.g., nickel chelate). In another embodiment, the adsorbent comprises an antibody that specifically binds to the marker.

Also disclosed is a purified protein selected from the protein markers of Marker Set 1 or Marker Set 2. In one embodiment the purified protein is produced by a process comprising fractionating a sample comprising the marker or markers by size exclusion chromatography and collecting a fraction that includes the marker or markers; and/or fractionating a sample comprising the marker or markers by anion exchange chromatography and collecting a fraction that includes the marker or markers.

Also disclosed is a kit comprising: (1) an adsorbent attached to a substrate, wherein the adsorbent retains a protein marker selected Marker Set 1 or Marker Set 2; and (2) instructions to detect the marker or markers by contacting a sample with the adsorbent and detecting the marker or markers retained by the adsorbent. In one embodiment of this invention the substrate is a probe for a gas phase ion spectrometer having a surface on which the adsorbent is attached. The adsorbent can be, *e.g.,* a hydrophilic adsorbent (*e.g*., silicon oxide)). In another embodiment the kit further comprises (1) an eluant wherein the marker or markers are retained on the adsorbent when washed with the eluent, or (2) instructions to wash adsorbent with the eluent after contacting the adsorbent with the marker or markers.

### BRIEF DESCRIPTION OF THE DRAWINGS

FIG 1 is a chart showing the detection of the set of seven markers of Marker Set 1 (SP1-SP7) in seminal plasma in samples from patients with each of prostate cancer (CAP), benign prostate hyperplasia (BPH) and a normal, or negative diagnosis (NR). The apparent molecular weights by mass spectrometry also are given with confidence intervals.
FIGS 2A-2G present typical mass spectrometry traces from a normal phase surface of markers SP1 to SP7 comparing prostate cancer (CAP), benign prostate hyperplasia (BPH) and normal (NP).
FIG 3 is a chart showing the detection of the set of four markers of Marker Set 1 (CL1-CL4) in prostate epithelial cell lysates isolated by laser capture microdissection in samples from patients with each of prostate cancer (CAP), benign prostate hyperplasia (BPH) and a normal, or negative diagnosis (NPr). The apparent molecular weights by mass spectrometry also are given with confidence intervals.
FIGS 4A-4C presents typical mass spectrometry traces from anion exchange or nickel chelate surfaces of Markers CL1 to CL4 in prostate cancer (CAP or CPA), benign prostate hyperplasia (BPH) and normal (NR). FIG 4A shows Marker CL1. FIG 4B shows Marker CL2. FIG 4C shows Markers CL3 and CL4.
FIG 5A is a chart of relative abundance of Marker CL3 and Marker CL4 in individual CaP, BPH and normal samples. FIG 5B is a bar graph of the mean ratio of CL4:CL3 in normal (NP), BPH and CaP.
FIG 6 depicts a probe comprising a substrate 101 and discontinuous spots of adsorbents **102**. The probe is removably insertable into a gas phase ion spectrometer. Each spot is addressable by an energy source for desorbing the analyte.

### DESCRIPTION OF THE SPECIFIC EMBODIMENTS

### I. DEFINITIONS

Unless defined otherwise, all technical and scientific terms used herein have the meaning commonly understood by a person skilled in the art to which this invention belongs. The following references provide one of skill with a general definition of many of the terms used in this invention: Singleton *et al.*, *Dictionary of Microbiology and Molecular Biology* (2nd ed. 1994); *The Cambridge Dictionary of Science and Technology* (Walker ed., 1988); *The Glossary of Genetics,* 5th Ed., R. Rieger *et al.* (eds.), Springer Verlag (1991); and Hale & Marham, *The Harper Collins Dictionary of Biology* (1991). As used herein, the following terms have the meanings ascribed to them unless specified otherwise.

"Marker" in the context of the present invention refers to an organic biomolecule, particularly a polypeptide, which is differentially present in a sample taken from patients having prostate cancer or benign prostate hyperplasia as compared to a comparable sample taken from subjects who do not have prostate cancer (*e.g*., negative diagnosis, normal or healthy subject). For examples, a marker can be a polypeptide (having a particular apparent molecular weight) which is present at an elevated or decreased level in samples of prostate cancer patients compared to samples of patients with a negative diagnosis.

"Organic biomolecule" refers to an organic molecule of biological origin, *e.g*., steroids, amino acids, nucleotides, sugars, polypeptides, polynucleotides, complex carbohydrates or lipids.

The phrase "differentially present" refers to differences in the quantity of a polypeptide (of a particular apparent molecular weight) present in a sample taken from patients having prostate cancer as compared to a comparable sample taken from patients who do not have prostate cancer (*e.g*., have benign prostate hyperplasia). A polypeptide is differentially present between the two samples if the amount of the polypeptide in one sample is significantly different from the amount of the polypeptide in the other sample. For example, a polypeptide is differentially present between the two samples if it is present at least about 150%, at least about 200%, at least about 500% or at least about 1000% greater than it is present in the other sample, or if it is detectable in one sample and not detectable in the other. Any polypeptides that are differentially present in samples taken from prostate cancer patients as compared to subjects who do not have prostate cancer (*e.g*., benign prostate hyperplasia patients) can be used as markers.

"Diagnostic" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

A "test amount" of a marker refers to an amount of a marker present in a sample being tested. A test amount can be either in absolute amount (*e.g*., µg/ml) or a relative amount (*e.g*., relative intensity of signals).

A "diagnostic amount" of a marker refers to an amount of a marker in a subject's sample that is consistent with a diagnosis of prostate cancer. A diagnostic amount can be either in absolute amount (*e.g.,* µg/ml) or a relative amount (*e.g.,* relative intensity of signals).

A "control amount" of a marker can be any amount or a range of amount which is to be compared against a test amount of a marker. For example, a control amount of a marker can be the amount of a marker (*e.g*., seminal basic protein) in a prostate cancer patient, a BPH patient or a person without prostate cancer or BPH. A control amount can be either in absolute amount (*e.g.,* µg/ml) or a relative amount (*e.g*., relative intensity of signals).

"Probe" refers to a device that, when positionally engaged in an interrogatable relationship to an ionization source, *e.g*., a laser desorption/ionization source, and in concurrent communication at atmospheric or subatmospheric pressure with a detector of a gas phase ion spectrometer, can be used to introduce ions derived from an analyte into the spectrometer. As used herein, the "probe" is typically reversibly engageable (*e.g.*, removably insertable) with a probe interface that positions the probe in an interrogatable relationship with the ionization source and in communication with the detector. A probe will generally comprise a substrate comprising a sample presenting surface on which an analyte is presented to the ionization source.

"Substrate" or "probe substrate" refers to a solid phase onto which an adsorbent can be provided (*e.g*., by attachment, deposition, *etc*.)

"Ionization source" refers to a device that directs ionizing energy to a sample presenting surface of a probe to desorb and ionize analytes from the probe surface into the gas phase. The preferred ionization source is a laser (used in laser desorption/ionization), in particular, nitrogen lasers, Nd-Yag lasers and other pulsed laser sources. Other ionization sources include fast atoms (used in fast atom bombardment), plasma energy (used in plasma desorption) and primary ions generating secondary ions (used in secondary ion mass spectrometry).

"Gas phase ion spectrometer" refers to an apparatus that detects gas phase ions. In the context of this invention, gas phase ion spectrometers include an ionization source used to generate the gas phase ions. Gas phase ion spectrometers include, for example, mass spectrometers, ion mobility spectrometers, and total ion current measuring devices.

"Gas phase ion spectrometry" refers to a method comprising employing an ionization source to generate gas phase ions from an analyte presented on a sample presenting surface of a probe and detecting the gas phase ions with a gas phase ion spectrometer.

"Mass spectrometer" refers to a gas phase ion spectrometer that measures a parameter which can be translated into mass-to-charge ratios of gas phase ions. Mass spectrometers generally include an inlet system, an ionization source, an ion optic assembly, a mass analyzer, and a detector. Examples of mass spectrometers are time-of-flight, magnetic sector, quadrapole filter, ion trap, ion cyclotron resonance, electrostatic sector analyzer and hybrids of these.

"Mass spectrometry" refers to a method comprising employing an ionization source to generate gas phase ions from an analyte presented on a sample presenting surface of a probe and detecting the gas phase ions with a mass spectrometer.

"Laser desorption mass spectrometer" refers to a mass spectrometer which uses laser as a means to desorb, volatilize and ionize an analyte.

"Adsorbent" refers to any material capable of adsorbing a marker. The term "adsorbent" is used herein to refer both to a single material ("monoplex adsorbent") (*e.g.*, a compound or functional group) to which the marker is exposed, and to a plurality of different materials ("multiplex adsorbent") to which the marker is exposed. The adsorbent materials in a multiplex adsorbent are referred to as "adsorbent species." For example, an addressable location on a probe substrate can comprise a multiplex adsorbent characterized by many different adsorbent species (*e.g*., anion exchange materials, metal chelators, or antibodies), having different binding characteristics. Substrate material itself can also contribute to adsorbing a marker and may be considered part of an "adsorbent."

"Adsorption" or "retention" refers to the detectable binding between an absorbent and a marker either before or after washing with an eluant (selectivity threshold modifier) or a washing solution.

"Eluant" or "washing solution" refers to an agent that can be used to mediate adsorption of a marker to an adsorbent. Eluants and washing solutions also are referred to as "selectivity threshold modifiers." Eluants and washing solutions can be used to wash and remove unbound materials from the probe substrate surface.

"Resolve," "resolution," or "resolution of marker" refers to the detection of at least one marker in a sample. Resolution includes the detection of a plurality of markers in a sample by separation and subsequent differential detection. Resolution does not require the complete separation of a marker from all other markers in a mixture. Rather, any separation that allows the distinction between at least two markers suffices.

"Detect" refers to identifying the presence, absence or amount of the object to be detected.

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, *e.g*., by the addition of carbohydrate residues to form glycoproteins. The terms "polypeptide," "peptide" and "protein" include glycoproteins, as well as non-glycoproteins.

"Detectable moiety" or a "label" refers to a composition detectable by spectroscopic, photochemical, biochemical, immunochemical, or chemical means. For example, useful labels include ³²P, ³⁵S, fluorescent dyes, electron-dense reagents, enzymes (*e.g*., as commonly used in an ELISA), biotin-streptavadin, dioxigenin, haptens and proteins for which antisera or monoclonal antibodies are available, or nucleic acid molecules with a sequence complementary to a target. The detectable moiety often generates a measurable signal, such as a radioactive, chromogenic, or fluorescent signal, that can be used to quantify the amount of bound detectable moiety in a sample. The detectable moiety can be incorporated in or attached to a primer or probe either covalently, or through ionic, van der Waals or hydrogen bonds, *e.g*., incorporation of radioactive nucleotides, or biotinylated nucleotides that are recognized by streptavadin. The detectable moiety may be directly or indirectly detectable. Indirect detection can involve the binding of a second directly or indirectly detectable moiety to the detectable moiety. For example, the detectable moiety can be the ligand of a binding partner, such as biotin, which is a binding partner for streptavadin, or a nucleotide sequence, which is the binding partner for a complementary sequence, to which it can specifically hybridize. The binding partner may itselfbe directly detectable, for example, an antibody may be itself labeled with a fluorescent molecule. The binding partner also may be indirectly detectable, for example, a nucleic acid having a complementary nucleotide sequence can be a part of a branched DNA molecule that is in turn detectable through hybridization with other labeled nucleic acid molecules. (*See, e.g.,* P. D. Fahrlander and A. Klausner, *Bio*/*Technology* 6:1165 (1988)). Quantitation of the signal is achieved by, *e.g.,* scintillation counting, densitometry, or flow cytometry.

"Antibody" refers to a polypeptide ligand substantially encoded by an immunoglobulin gene or immunoglobulin genes, or fragments thereof, which specifically binds and recognizes an epitope (*e.g*., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, *e.g*., as intact immunoglobulins or as a number of well characterized fragments produced by digestion with various peptidases. This includes, *e.g*., Fab' and F(ab)'₂ fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region.

"Immunoassay" is an assay that uses an antibody to specifically bind an antigen. The immunoassay is characterized by the use of specific binding properties of a particular antibody to isolate, target, and/or quantify the antigen.

The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. Specific binding to an antibody under such conditions may require an antibody that is selected for its specificity for a particular protein. For example, polyclonal antibodies raised to seminal basic protein from specific species such as rat, mouse, or human can be selected to obtain only those polyclonal antibodies that are specifically immunoreactive with seminal basic protein and not with other proteins, except for polymorphic variants and alleles of seminal basic protein. This selection may be achieved by subtracting out antibodies that cross-react with seminal basic protein molecules from other species. A variety of immunoassay formats may be used to select antibodies specifically immunoreactive with a particular protein. For example, solid-phase ELISA immunoassays are routinely used to select antibodies specifically immunoreactive with a protein (*see, e.g.,* Harlow & Lane, *Antibodies, A Laboratory Manual* (1988), for a description of immunoassay formats and conditions that can be used to determine specific immunoreactivity). Typically a specific or selective reaction will be at least twice background signal or noise and more typically more than 10 to 100 times background.

### II. GAS PHASE ION SPECTROMETRY

In one aspect, the invention provides methods for detecting markers which are differentially present in samples from patients with prostate cancer, benign prostate hyperplasia and with negative diagnoses. Any one or combination of markers described are within the scope of this aspect of this invention and can be detected. The methods for detecting these markers have many applications. For example, one marker or combination of markers can be measured to differentiate between prostate cancer and BPH, and thus are useful as an aid in the diagnosis of prostate cancer in a patient. In another example, the present methods for detecting these markers can be applied to *in vitro* prostate cancer cells or *in vivo* animal models for prostate cancer to assay for and identify compounds that modulate expression of these markers. In one embodiment of the detection method, the marker or markers are detected by gas phase ion spectrometry. In a preferred embodiment, the marker or markers are detected by mass spectrometry and, in particular, laser desorption mass spectrometry.

Probes suitable for use in the invention are described in, *e.g*., U.S. Patent 5,617,060 (Hutchens and Yip) and WO 98/59360 (Hutchens and Yip). In one embodiment, a substrate comprising an adsorbent can be in the form of a probe, which is removably insertable into a gas phase ion spectrometer. For example, a substrate can be in the form of a strip with adsorbents on its surface. In another embodiment, a substrate comprising an adsorbent can be positioned onto another substrate to form a probe, which is removably insertable into a gas phase ion spectrometer. For example, a substrate comprising an adsorbent can be a solid phase, such as a polymeric or glass bead with a functional group for binding a marker, which can be subsequently positioned on a second substrate to form a probe. For example, the second substrate can be in the form of a strip, or a plate having a series of wells at a predetermined addressable locations. One advantage of this embodiment is that the marker can be adsorbed to the first substrate in one physical context, and transferred to the second substrate, which can then be submitted for analysis by gas phase ion spectrometry. The probe can be in any shape as long as it is removably insertable into a gas phase ion spectrometer.

The probe can also be adapted for use with inlet systems and detectors of a gas phase ion spectrometer. For example, the probe can be adapted for mounting in a horizontally, vertically and/or rotationally translatable carriage that moves the probe to a successive position without requiring repositioning of the probe by hand.

The probe substrate is preferably made of a material that is capable of supporting adsorbents. For example, the probe substrate material can include, but is not limited to, insulating materials (*e.g.*, glass, ceramic), semi-insulating materials (*e.g.*, silicon wafers), or electrically conducting materials (*e.g*., metals, such as nickel, brass, steel, aluminum, gold, or electrically conductive polymers), organic polymers, biopolymers, or any combinations thereof

The probe substrate surface can be conditioned to bind markers. For example, in one embodiment, the surface of the probe substrate can be conditioned (*e.g*., chemically or mechanically such as roughening) to place adsorbents on the surface. The adsorbent comprises functional groups for binding with a marker. In some embodiments, the substrate material itself can also contribute to adsorbent properties and may be considered part of an "adsorbent."

Any number of different adsorbents can be used as long as they have binding characteristics suitable for binding the markers of the present invention. The adsorbents can comprise a hydrophobic group, a hydrophilic group, a cationic group, an anionic group, a metal ion chelating group, or antibodies which specifically bind to antigens, or a combination thereof (sometimes referred to as "a mixed mode" adsorbent). Exemplary adsorbents comprising a hydrophobic group include matrices having aliphatic hydrocarbons, *e.g.*, C₁-C₁₈ aliphatic hydrocarbons and matrices having aromatic hydrocarbon functional group such as phenyl groups. Exemplary adsorbents comprising a hydrophilic group include silicon oxide (*i.e*., glass), or hydrophilic polymers such as polyethylene glycol, dextran, agarose, or cellulose. Exemplary adsorbents comprising a cationic group include matrices of secondary, tertiary or quaternary amines. Exemplary adsorbents comprising an anionic group include matrices of sulfate anions (SO₃⁻) and matrices of carboxylate anions (*i.e.*, COO⁻) or phosphate anions (OPO₃⁻). Exemplary adsorbents comprising metal chelating groups include organic molecules that have one or more electron donor groups which form coordinate covalent bonds with metal ions, such as copper, nickel, cobalt, zinc, iron, and other metal ions such as aluminum and calcium. Exemplary adsorbents comprising an antibody include antibodies that are specific for any one of the markers provided herein. In preferred embodiments, adsorbents are substantially similar to or the same as the adsorbents which were used to enrich and identify the markers.

One useful chip is the Normal Phase chip, available from Ciphergen Biosystems, Inc. (Palo Alto, CA). The normal phase chip has a hydrophilic adsorbent comprising silicon oxide (SiO₂) on the substrate surface. Silicon oxide can be applied to the surface by any of a number of well known methods. These methods include, for example, vapor deposition, e.g., sputter coating. A preferred thickness for such a probe is about 9000 Angstroms.

Another useful chip is the SAX1 ProteinChip™ made by Ciphergen Biosystems, Inc. in Palo Alto, CA. The SAX1 protein chips are fabricated from SiO₂ coated aluminum substrates. In the process, a suspension of quaternary ammonium polystryenemicrospheres in distilled water is deposited onto the surface of the chip (1 mL/spot, two times). After air drying (room temperature, 5 minutes), the chip is rinsed with deionized water and air dried again (room temperature, 5 minutes).

Another useful chip is the IMAC3 (Immobilized Metal Affinity Capture, nitrilotriacetic acid on surface) chip, also available from Ciphergen Biosystems, Inc. The chips are produced as follows: 5-Methacylamido-2-(N,N-biscarboxymethaylamino)pentanoic acid (7.5 wt%),Acryloyltri-(hydroxymethyl)methylamine (7.5 wt%) and N,N'-methylenebisacrylamide (0.4 wt%) are photo-polymerized using-(-)riboflavin (0.02 wt%) as a photo-initiator. The monomer solution is deposited onto a rough etched, glass coated substrate (0.4 mL, twice) and irradiated for 5 minutes with a near UV exposure system (Hg short arc lamp, 20 mW/cm2 at 365 nm). The surface is washed with a solution of sodium chloride (1 M) and then washed twice with deionized water.

The IMAC3 with Ni(II) is activated as follows. The surface is treated with a solution of NiSO₄ (50 mM, 10 mL/spot) and mixed on a high frequency mixer for 10 minutes. After removing the NiSO₄ solution, the treatment process is repeated. Finally, the surface is washed with a stream of deionized water (15 sec/chip).

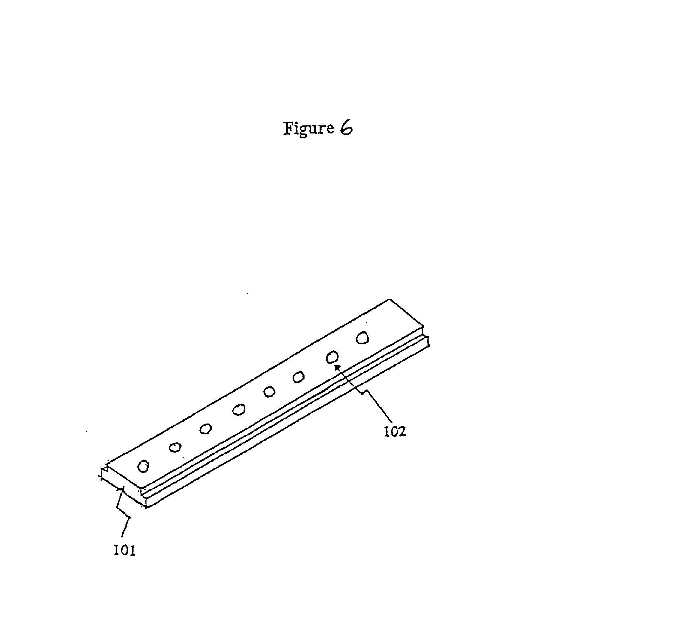

Adsorbents can be placed on the probe substrate in continuous or discontinuous patterns. If continuous, one or more adsorbents can be placed on the substrate surface. If multiple types of adsorbents are used, the substrate surface can be coated such that one or more binding characteristics vary in one or two-dimensional gradient. If discontinuous, plural adsorbents can be placed in predetermined addressable locations on the substrate surface. The addressable locations can be arranged in any pattern, but are preferably in regular pattern, such as lines, orthogonal arrays, or regular curves (*e.g*., circles). Each addressable location may comprise the same or different adsorbent. In FIG 6, a probe comprising discontinuous spots of adsorbents is shown. The spots are "addressable" in that during mass spectrometry, an energy source, such as a laser, is directed to, or "addresses" each spot differentially to desorb the analyte.

The probes can be produced using any suitable methods depending on the selection of substrate materials and/or adsorbents. For example, the surface of a metal substrate can be coated with a material that allows derivitization of the metal surface. More specifically, a metal surface can be coated with silicon oxide, titanium oxide or gold. Then surface can be derivatized with a bifunctional linker, one end of which can covalently bind with a functional group on the surface and the other end of which can be further derivatized with groups that function as an adsorbent. In another example, a porous silicon surface generated from crystalline silicon can be chemically modified to include adsorbents for binding markers. In yet another example, adsorbents with a hydrogel backbone can be formed directly on the substrate surface by *in situ* polymerizing a monomer solution which comprises, *e.g*., substituted acrylamide monomers, substituted acrylate monomers, or derivatives thereof comprising a functional group of choice as an adsorbent.

The probe substrate comprising an adsorbent contacts a sample. The sample is preferably a biological fluid sample. Examples of biological fluid samples include blood, serum, urine, prostatic fluid, seminal fluid, semen, seminal plasma and prostate tissue (e.g., epithelial tissue, including extracts thereof).

The sample can be solubilized in or admixed with an eluant. The probe substrate comprising an adsorbent then contacts the solution using any techniques including bathing, soaking, dipping, spraying, washing over, or pipetting, *etc*. Generally, a volume of sample containing from a few attomoles to 100 picomoles of marker in about 1 µl to 500 µl is sufficient for binding to the adsorbent.

The sample can contact the probe substrate comprising an adsorbent for a period of time sufficient to allow the marker to bind to the adsorbent. Typically, the sample and the substrate comprising the adsorbent are contacted for a period of between about 30 seconds and about 12 hours, and preferably, between about 30 seconds and about 15 minutes.

The temperature at which the sample contacts the probe substrate comprising an adsorbent can be a function of the particular sample and the selected probe. Typically, the sample is contacted to the probe substrate under ambient temperature and pressure conditions. For some samples, however, modified temperature (typically 4°C through 37°C), and pressure conditions can be desirable, which conditions are detcrminable by those skilled in the art.

After the probe substrate comprising an adsorbent contacts the sample or sample solution, it is preferred that unbound materials on the probe substrate surface are washed out so that only the bound materials remain on the substrate surface. Washing a probe substrate surface can be accomplished by, *e.g*., bathing, soaking, dipping, rinsing, spraying, or washing the substrate surface with an eluant or a washing solution. A microfluidics process is preferably used when a washing solution such as an eluant is introduced to small spots of adsorbents on the probe. Typically, the washing solution can be at a temperature of between 0°C and 100°C, preferably between 4°C and 37°C.

Any suitable washing solutions or eluants can be used to wash the probe substrate surface. For example, organic solutions or aqueous solutions can be used. Preferably, an aqueous solution is used. Exemplary aqueous solutions include a HEPES buffer, a Tris buffer, a phosphate buffered saline, *etc.* The selection of a particular washing solution or an eluant is dependent on other experimental conditions (e.g., types of adsorbents used or markers to be detected), and can be determined by those of skill in the art. For example, if a probe comprising a hydrophobic group and a sulfonate group as adsorbents (*e.g*., SAX1 ProteinChip^{™} array) is used, then an aqueous solution, such as a HEPES buffer, may be preferred. In another example, if a probe comprising a metal binding group as an adsorbent (*e.g*., Ni(II) ProteinChip^{™} array) is used, then an aqueous solution, such as a phosphate buffered saline, may be preferred. In yet another example, if a probe comprising a hydrophobic group (*e.g*., H4 ProteinChip^{™}) is used, then water may be preferred as a washing solution.

An energy absorbing molecule (*e.g*., in solution) can be applied to markers or other substances bound on the probe substrate surface. Spraying, pipetting, or dipping can be used. This can be done after unbound materials are washed off of the probe substrate surface. An energy absorbing molecule refers to a molecule that absorbs energy from an energy source in a gas phase ion spectrometer, thereby assisting desorption of markers or other substances from a probe surface. Exemplary energy absorbing molecules include cinnamic acid derivatives, sinapinic acid and dihydroxybenzoic acid.

After the marker is bound to the probe, it is detected using gas phase ion spectrometry. Markers or other substances bound to the adsorbents on the probes can be analyzed using a gas phase ion spectrometer. The quantity and characteristics of the marker can be determined using gas phase ion spectrometry. Other substances in addition to the marker of interest can also be detected by gas phase ion spectrometry.

In one embodiment, a mass spectrometer can be used to detect markers on the probe. In a typical mass spectrometer, a probe with a marker is introduced into an inlet system of the mass spectrometer. The analyte is then desorbed by a desorption source such as a laser, fast atom bombardment, or high energy plasma. The generated desorbed, volatilized species consist of preformed ions or neutrals which are ionized as a direct consequence of the desorption event. Generated ions are collected by an ion optic assembly, and then a mass analyzer disperses and analyzes the passing ions. The ions exiting the mass analyzer are detected by a detector. The detector then translates information of the detected ions into mass-to-charge ratios. Detection of the presence of a marker or other substances will typically involve detection of signal intensity. This, in turn, can reflect the quantity and character of a marker bound to the probe.

In a preferred embodiment, a laser desorption time-of-flight mass spectrometer is used with the probe of the present invention. In laser desorption mass spectrometry, a probe with a bound analyte is introduced into an inlet system. The analyte is desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of molecules of specific mass to charge ratio. As any person skilled in the art understands, any of these components of the laser desorption time-of-flight mass spectrometer can be combined with other components described herein in the assembly of mass spectrometer that employs various means of desorption, acceleration, detection, measurement of time, *etc.*

In another embodiment, an ion mobility spectrometer can be used to detect and characterize a marker. The principle of ion mobility spectrometry is based on different mobility of ions. Specifically, ions of a sample produced by ionization move at different rates, due to their difference in, *e.g*., mass, charge, or shape, through a tube under the influence of an electric field. The ions (typically in the form of a current) are registered at the detector which can then be used to identify a marker or other substances in the sample. One advantage of ion mobility spectrometry is that it can operate at atmospheric pressure.

In yet another embodiment, a total ion current measuring device can be used to detect and characterize markers. This device can be used when the probe has a surface chemistry that allows only a single type of marker to be bound. When a single type of marker is bound on the probe, the total current generated from the ionized marker reflects the nature of the marker. The total ion current produced by the marker can then be compared to stored total ion current of known compounds. Characteristics of the marker can then be determined.

Data generated by desorption and detection of markers can be analyzed with the use of a programmable digital computer. The computer program generally contains a readable medium that stores codes. Certain code can be devoted to memory that includes the location of each feature on a probe, the identity of the adsorbent at that feature and the elution conditions used to wash the adsorbent. Using this information, the program can then identify the set of features on the probe defining certain selectivity characteristics (*e.g*., types of adsorbent and eluants used). The computer also contains code that receives as input, data on the strength of the signal at various molecular masses received from a particular addressable location on the probe. This data can indicate the number of markers detected, optionally including the strength of the signal and the determined molecular mass for each marker detected.

Data analysis can include the steps of determining signal strength (*e.g*., height of peaks) of a marker detected and removing "outerliers" (data deviating from a predetermined statistical distribution). For example, the observed peaks can be normalized, a process whereby the height of each peak relative to some reference is calculated. For example, a reference can be background noise generated by instrument and chemicals (*e.g*., energy absorbing molecule) which is set as zero in the scale. Then the signal strength detected for each marker or other substances can be displayed in the form of relative intensities in the scale desired (*e.g*., 100). Alternatively, a standard may be admitted with the sample so that a peak from the standard can be used as a reference to calculate relative intensities of the signals observed for each marker or other markers detected.

The computer can transform the resulting data into various formats for displaying. In one format, referred to as "spectrum view or retentate map," a standard spectral view can be displayed, wherein the view depicts the quantity of marker reaching the detector at each particular molecular weight. In another format, referred to as "peak map," only the peak height and mass information are retained from the spectrum view, yielding a cleaner image and enabling markers with nearly identical molecular weights to be more easily seen. In yet another format, referred to as "gel view," each mass from the peak view can be converted into a grayscale image based on the height of each peak, resulting in an appearance similar to bands on electrophoretic gels. In yet another format, referred to as "3-D overlays," several spectra can be overlaid to study subtle changes in relative peak heights. In yet another format, referred to as "difference map view," two or more spectra can be compared, conveniently highlighting unique markers and markers which are up- or down-regulated between samples. Marker profiles (spectra) from any two samples may be compared visually.

### III. METHODS OF DETECTING MARKERS

### A. Characterization Of The Markers

Two sets of markers were identified that are useful in distinguishing prostate cancer, benign prostate hyperplasia and a negative diagnosis. A first set was identified in seminal plasma. A second set was identified in cell lysate from prostate epithelial tissue.

### 1. Marker Set:1 Seminal Plasma

A first set of markers useful for diagnosing prostate cancer or benign prostate hyperplasia has the following molecular weights as determined by mass spectrometry: Marker SP1: 9,402.68 ± 8.97 Da, Marker SP2: 26,155.30 ± 202.01 Da, Marker SP3: 54,979.27 ± 408.78 Da, Marker SP4: 9,752.30 ± 15.08 Da, Marker SP5: 87,66.93 ± 14.44 Da, Marker SP6: 62,77.97 ± 12.36 Da and Marker SP7:2,781.72 ± 4.41 Da.

These markers are further characterized as follows. Seminal plasma was first fractionated by size exclusion spin chromatography using a matrix that excludes proteins having molecular weight greater than about 30 kD. SP4 and SP5 are sufficiently abundant that they can be detected without further fractionation by SELDI mass spectrometry using a normal phase adsorbent chip. Fractions from the sizing column were subject to further fractionation by applying them to a strong anion exchange affinity spin column and eluting with an eluent of 20 mM Tris, 0.4 M NaCl, 0.1 % TritonX 100 using a pH gradient ranging from pH 9.0 to pH 4.0. Markers SP1 and SP2 eluted in a late fraction, indicating a low pI, around 5. Markers SP3 and SP7 eluted in early fractions, indicating a pI around 9. The markers were detected by SELDI mass spectrometry using a normal phase adsorbent chip.

### 2. Marker Set:2 Cell Lysate

A second set of markers useful for diagnosing prostate cancer, benign prostate hyperplasia and a negative diagnosis has the following molecular weights as determined by mass spectrometry: Marker CL1: 8,494.30 ± 10.24 Da, Marker CL2: 9,614.62 ± 52.19 Da, Marker CL3: 28,472 ± 127.40 Da, and Marker CL4: 33,386.85 ± 160.47 Da.

These markers are further characterized as follows: Cell lysate from prostate epithelial cells isolated by laser capture microdissection was fractionated and analyzed by retentate chromatography. In this method, test samples were applied to a substrate comprising one of two attached adsorbents - a strong anion exchange adsorbent or a nickel chelate adsorbent. The adsorbents were washed with a first eluent of 20 mM Tris, pH 7.5, 0.1% TritonX 100, 0.5M NaCl to allow binding of proteins to the chip, and with a second eluent of water to remove non-bound materials. Markers retained on the adsorbents were detected by SELDI mass spectrometry. The four markers could be resolved by either of the two adsorbents. However, markers CL1 and CL2 were resolved better on the anion exchange adsorbent, and markers CL3 and CL4 were resolved better on the metal chelate adsorbent. Because the markers bound to an anion exchange adsorbent at neutral pH, this implies that they are negatively charged at neutral pH.

### B. Sample Sources For Markers

These markers can be detected in many different samples derived from a patient or subject. The sample is preferably a biological fluid sample. Examples of biological fluid samples useful in this invention include blood, serum, urine, prostatic fluid, seminal fluid, semen, seminal plasma and prostate tissue (*e.g*., epithelial tissue, including extracts thereof).

Because the markers of Marker Set 1 were discovered in seminal plasma, seminal plasma is a preferred sample source for the methods of this invention. Moreover, testing a seminal plasma sample does not require an invasive procedure, such as inserting a needle into a patient.

Because the markers of Marker Set 2 were discovered in cell lysate from prostate epithelium, this tissue is a preferred sample source for the methods of this invention. More particularly, the samples were collected by laser capture microdissection of this tissue. Therefore, this is a preferred method of obtaining sample for the methods of this invention.

### C. Detection of Markers

After a sample is obtained, any suitable method can be used to detect the marker in a sample from a subject being tested. For example, gas phase ion spectrometry or an immunoassay can be used.

### 1. Gas Phase Ion Spectrometry

In a preferred embodiment, the markers of this invention are detected using gas phase ion spectrometry and, more preferably, using mass spectrometry and, still more preferably, using surface-enhanced laser desorption/ionization mass spectrometry ("SELDI"). SELDI is an improved method of gas phase ion spectrometry for biomolecules. In SELDI, the surface on which the analyte is applied plays an active role in the analyte capture, desorption and/or desorption. One popular method of gas phase ion spectrometry for biomolecules is MALDI (matrix-assisted laser desorption/ionization) mass spectrometry. In MALDI, the analyte is typically mixed with a matrix material that, upon drying, forms crystals that capture the analyte. The matrix material absorbs energy from the energy source which otherwise would fragment the bimolecular analytes.

### a) Preparation of sample

### (i) Pre-fractionation

In one embodiment, the sample can be pre-fractionated before being subjected to gas phase ion spectrometry. Pre-fractionation has the advantage of providing a less complex sample for analysis. On the other hand, it introduces an extra step in the analytic process which could be unattractive in, for example, a clinical setting. Samples can be pre-fractionated by any means known in the art, including, without limitation, size fractionation and chromatographic fractionation.

In one embodiment, seminal plasma samples can be pre-fractionated before analysis by gas-phase ion spectrometry. A preferred method of fraction includes a first fractionation by gel exclusion chromatography. Sizing columns which exclude molecules whose molecular mass is greater than 30 kD are particularly useful for this. Fractions of various sizes then can be examined directly or subjected to a second fractionation step based on anion exchange chromatography. Using an anion exchange Q spin column, markers SP1, SP2, SP3 and SP7 can be eluted using a low strength buffer (*e.g.*, about 10 mM to 50 mM Tris, HEPES or PBS) with salt at low to medium concentration (e.g., about 0.1 M to 0.6 M) and a non-ionic detergent at low concentration (*e.g*., TritonX 100 at about 0.05 to 0.2%). A particularly useful buffer is 20 mM Tris, 0.5 M NaCl and 0.1% TritonX 100. Markers SP1 and SP2 will elute at about pH 5 and markers SP3 and SP7 will elute at about pH 9. In one embodiment, the markers are eluted using a pH gradient.

### (ii) Retentate Chromatography

In another embodiment, the sample is fractionated on a bio-chromatographic chip by retentate chromatography before gas phase ion spectrometry. A preferred chip is the Protein Chip^{™} available from Ciphergen Biosystems, Inc. (Palo Alto, CA). As described above, the chip or probe is adapted for use in a mass spectrometer. The chip comprises an adsorbent attached to its surface. This adsorbent can function, in certain applications, as an *in situ* chromatography resin. In basic operation, the sample is applied to the adsorbent in an eluent solution. Molecules for which the adsorbent has affinity under the wash condition bind to the adsorbent. Molecules that do not bind to the adsorbent are removed with the wash. The adsorbent can be further washed under various levels of stringency so that analytes are retained or eluted to an appropriate level for analysis. Then, an energy absorbing molecule can be added to the adsorbent spot to further facilitate desorption and ionization. The analyte is detected by desorption from the adsorbent, ionization and direct detection by a detector. Thus, retentate chromatography differs from traditional chromatography in that the analyte retained by the affinity material is detected, whereas in traditional chromatography, material that is eluted from the affinity material is detected.

A useful adsorbent for resolving the markers of Marker Set 1 is a hydrophilic adsorbent and, in particular, a silicon oxide (normal phase) adsorbent. Seminal plasma pre-fractionated by sizing followed by anion exchange chromatography can be applied directly to the surface of a normal phase adsorbent without the need for washing. Instead, the sample is allowed to dry on the surface. This is because the pre-fractionation steps remove sufficient numbers of proteins that the markers can be resolved without further fractionation. Then, an energy absorbing molecule can be applied.

Another useful adsorbent for at least Markers SP1 and SP2 is an anion exchange adsorbent. This is because these proteins have a pI around 5. Therefore, they are expected to bind to an anion exchange adsorbent when washed with a neutral pH eluent. Markers SP3 and SP7 have a pI around 9. Therefore, they are expected to be positively charged at neutral pH. Accordingly, these markers also can be resolved on a cationic adsorbent washed with a pH neutral buffer.

A useful adsorbent for resolving the markers of Marker Set 2 is an anion exchange adsorbent. This surface is usefully washed with a pH neutral buffer comprising low to medium salt and low concentration of non-ionic detergent. A preferred surface is SAX-1 from Ciphergen Biosystems, Inc. This surface is a strong anion exchange resin comprising quaternary ammonium ions.

Another useful adsorbent for the markers of Marker Set 2 is a metal chelate adsorbent and, in particular, nickel. This surface also is usefully washed with a pH neutral buffer comprising low to medium salt and low concentration of non-ionic detergent. A preferred surface is IMAC3 from Ciphergen Biosystems, Inc. IMAC3 comprises a nickel chelate adsorbent.

Another useful adsorbent for any of the markers of this invention is an antibody that specifically binds the marker. Chips comprising antibodies that bind to one or more markers are particularly useful for removing non-markers which do not bind to the antibodies and which function as "noise" in the detection process.

As will be evident to anyone skilled in the art, different markers may be more easily resolved using different combinations of adsorbents and eluants.

### (iii) MALDI

In another embodiment, the sample can be mixed with a matrix material and analyzed by traditional MALDI. In this case, the sample/matrix mixture is applied to the surface of an inert mass spectrometer probe. MALDI methods almost certainly will require purification before use - the resolving power of MALDI is limited by the complexity of the analytes in the sample.

### (iv) Modification of Marker Before Analysis

In another embodiment, the markers are modified before detection in order to alter their molecular weight. These methods may decrease ambiguity of detection. For example, the markers may be subject to proteolytic digestion before analysis. Any protease can be used. Proteases such as trypsin, that are likely to cleave the markers into a discrete number of fragments are particularly useful. The fragments that result from digestion function as a fingerprint for the markers, thereby enabling their detection indirectly. This is particularly useful where there are markers with similar molecular masses that might be confused for the marker in question. Also, proteolytic fragmentation is useful for high molecular weight markers because smaller markers are more easily resolved by mass spectrometry.

In another embodiment, the markers can be modified by the attachment of a tag of particular molecular weight that bind specifically to molecular markers, further distinguishing them.

### b) Performance of laser desorption/ionization mass spectrometry

After the marker is detected by gas phase ion spectrometry, a test amount of marker can be determined. For example, a signal is displayed at the molecular weight of the marker of interest. Based on the strength or magnitude of the displayed signal, the amount of marker in a sample being tested can be determined. It is noted that the test amount of marker in a sample need not be measured in absolute units, but can be in relative units as long as it can be compared qualitatively or quantitatively to a control amount of a marker. For example, the amount of the marker detected can be displayed in terms of relative intensity based on the background noise. Preferably, the test amount and the control amount of markers are measured under the same conditions.

If desired, the absolute amount of a marker can be determined by calibration. For example, a purified, known marker can be added in increasing amounts to different spots of adsorbents on the probe surface. Then peaks from each spot can be obtained and plotted in a graph against the concentration of seminal basic protein at each spot. From the peak intensity vs. concentration plot, the absolute amount of a marker in any sample being tested can be determined.

### 2. Immunoassay Detection

In another embodiment of the detection method, an immunoassay can be used to qualitatively or quantitatively detect and analyze markers in a sample. This method comprises: (a) providing an antibody that specifically binds to a marker; (b) contacting a sample with the antibody; and (c) detecting the presence of a complex of the antibody bound to the marker in the sample.

To prepare an antibody that specifically binds to a marker, purified markers or their nucleic acid sequences can be used. Nucleic acid and amino acid sequences for markers can be obtained by further characterization of these markers. For example, each marker can be peptide mapped with a number of enzymes (*e.g*., trypsin, V8 protease, *etc*.). The molecular weights of digestion fragments from each marker can be used to search the databases, such as SwissProt database, for sequences that will match the molecular weights of digestion fragments generated by various enzymes. Using this method, the nucleic acid and amino acid sequences of other markers can be identified if these markers are known proteins in the databases.

Alternatively, the proteins can be sequenced using protein ladder sequencing. Protein ladders can be generated by, for example, fragmenting the molecules and subjecting fragments to enzymatic digestion or other methods that sequentially remove a single amino acid from the end of the fragment. Methods of preparing protein ladders are described, for example, in International Publication WO 93/24834 (Chait et al.) and United States Patent 5,792,664 (Chait et al.). The ladder is then analyzed by mass spectrometry. The difference in the masses of the ladder fragments identify the amino acid removed from the end of the molecule.

If the markers are not known proteins in the databases, nucleic acid and amino acid sequences can be determined with knowledge of even a portion of the amino acid sequence of the marker. For example, degenerate probes can be made based on the N-terminal amino acid sequence of the marker. These probes can then be used to screen a genomic or cDNA library created from a sample from which a marker was initially detected. The positive clones can be identified, amplified, and their recombinant DNA sequences can be subcloned using techniques which are well known. *See, e.g., Current Protocols for Molecular Biology* (Ausubel *et al.,* Green Publishing Assoc. and Wiley-Interscience 1989) *and Molecular Cloning: A Laboratory Manual,* 2nd Ed. (Sambrook *et al.,* Cold Spring Harbor Laboratory, NY 1989).

Using the purified markers or their nucleic acid sequences, antibodies that specifically bind to a marker can be prepared using any suitable methods known in the art. *See, e.g.,* Coligan, *Current Protocols in Immunology* (1991); Harlow & Lane, *Antibodies: A Laboratory Manual* (1988); Goding, *Monoclonal Antibodies: Principles and Practice* (2d ed. 1986); and Kohler & Milstein, *Nature* 256:495-497 (1975). Such techniques include, but are not limited to, antibody preparation by selection of antibodies from libraries of recombinant antibodies in phage or similar vectors, as well as preparation of polyclonal and monoclonal antibodies by immunizing rabbits or mice (*see, e.g.*, *Huse et al., Science* 246:1275-1281 (1989); Ward *et al., Nature* 341:544-546 (1989)).

After the antibody is provided, a marker can be detected and/or quantified using any of a number of well recognized immunological binding assays (*see, e.g.,* U.S. Patents 4,366,241; 4,376,110; 4,517,288; and 4,837,168). Useful assays include, for example, an enzyme immune assay (EIA) such as enzyme-linked immunosorbent assay (ELISA), a radioimmune assay (RIA), a Western blot assay, or a slot blot assay. For a review of the general immunoassays, *see also, Methods in Cell Biology: Antibodies in Cell Biology,* volume 37 (Asai, ed. 1993); *Basic and Clinical Immunology* (Stites & Terr, eds., 7th ed. 1991).

Generally, a sample obtained from a subject can be contacted with the antibody that specifically binds the marker. Optionally, the antibody can be fixed to a solid support to facilitate washing and subsequent isolation of the complex, prior to contacting the antibody with a sample. Examples of solid supports include glass or plastic in the form of, *e.g.,* a microtiter plate, a stick, a bead, or a microbead. Antibodies can also be attached to a probe substrate or ProteinChip^{™} array described above. The sample is preferably a biological fluid sample taken from a subject. Examples of biological fluid samples include blood, serum, urine, prostatic fluid, seminal fluid, semen, seminal plasma and prostate tissue (*e.g*., epithelial tissue, including extracts thereof). In a preferred embodiment, the biological fluid comprises seminal plasma. The sample can be diluted with a suitable eluant before contacting the sample to the antibody.

After incubating the sample with antibodies, the mixture is washed and the antibody-marker complex formed can be detected. This can be accomplished by incubating the washed mixture with a detection reagent. This detection reagent may be, *e.g*., a second antibody which is labeled with a detectable label. Exemplary detectable labels include magnetic beads (*e.g*., DYNABEADS^{™}), fluorescent dyes, radiolabels, enzymes (*e.g.*, horse radish peroxide, alkaline phosphatase and others commonly used in an ELISA), and colorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume of solution, concentrations and the like. Usually the assays will be carried out at ambient temperature, although they can be conducted over a range of temperatures, such as 10°C to 40°C.

The immunoassay techniques are well-known in the art, and a general overview of the applicable technology can be found in Harlow & Lane, *supra.*

The immunoassay can be used to determine a test amount of a marker in a sample from a subject. First, a test amount of a marker in a sample can be detected using the immunoassay methods described above. If a marker is present in the sample, it will form an antibody-marker complex with an antibody that specifically binds the marker under suitable incubation conditions described above. The amount of an antibody-marker complex can be determined by comparing to a standard. As noted above, the test amount of marker need not be measured in absolute units, as long as the unit of measurement can be compared to a control amount.

### IV. METHODS OF DIAGNOSIS USING MARKERS

The markers in Marker Set 1 or Marker Set 2 can be used alone, in combination with other markers in the set, in combination with markers in either of the sets, or with other markers entirely (*e.g*., PSA or prostate specific membrane antigen) to aid in the diagnosis of prostate cancer, benign prostate hyperplasia or a negative diagnosis. Used together, they tend to provide more information for the diagnostician, increasing the percentage of true positive and true negative diagnoses and decreasing the percentage of false positive or false negative diagnoses, than a single marker alone.

In certain embodiments, it is useful to detect the presence or absence of a marker, without quantifying the amount of marker. This information is useful because the markers show differences in frequency of detection between CaP, BPH and negative diagnosis. In other embodiments, detecting the presence or absence of the markers can also involve quantifying the markers. This information is useful for several purposes. First, individual markers may be slightly detectable in certain states based on a weak peak, but easily detectable in other states based on a strong peak. Second, certain of the markers, for example CL3 and CL4, are frequently detected in all states, but their relative ratio differs dramatically between CaP and either of BPH and normal.

It is, of course, understood by diagnosticians that diagnostic tests are measured by their degree of specificity and sensitivity. Most diagnostic tests suffer from some imperfection in their ability to provide a positive result for every true positive diagnosis, as well as some imperfection in their ability to provide a negative result for every true negative diagnosis. However, tests which are not perfectly specific or sensitive are useful in diagnosis because they render certain diagnoses more likely than not. For example, marker SP1 is detected in 89% of all samples positive for CaP and only 22% of negative samples. Thus, detection of SP1 is useful in that it provides a probable diagnosis of CaP. Failure to detect SP1 also is useful in that it provides a probable negative diagnosis. Furthermore, used in combination with other markers, the diagnostician and increase their confidence in a CaP or negative diagnosis. With this in mind, the markers have frequency distributions as follows.

### A. Marker Set:1 Seminal Plasma

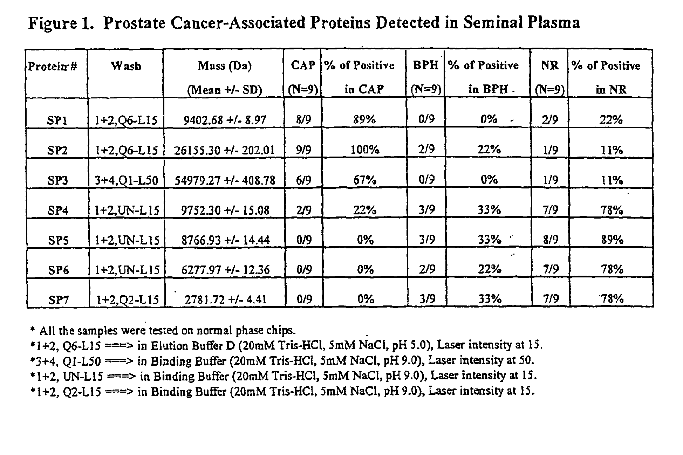

FIG 1 is a chart of the detection of markers SP1 to SP7 in CaP, BPH and negative diagnosis. Using this chart, the diagnostician can correlate the detection of one or more markers with the various diagnoses with varying levels of confidence. Compared to a negative diagnosis, SP1 is more frequently detected in CaP, and less frequently detected (*e.g*., undetected) in BPH. SP2 is more frequently detected in CaP and in BPH. SP3 is more frequently detected in CaP, and less frequently detected in BPH. SP4 is less frequently detected in both CaP and BPH. SP5, SP6 and SP7 are less frequently detected in (*e.g*., undetected) CaP and less frequently detected in BPH.

SP2 is the most discriminating single marker, detected in 100% of CaP samples, 0% of BPH samples and 11% ofnormal samples. SP1 and SP3 were not detected in BPH and their detection are useful for ruling out this condition. Thus, the detection of all three of SP1, SP2 and SP3 is highly correlated with CaP and negatively correlated with BPH. SP5, SP6 and SP7 were not detected in CaP. Therefore, their detection tends to rule out this diagnosis. Benign prostate hyperplasia is more difficult to positively diagnose. However, the condition is most strongly correlated with the detection of SP2, SP4, SP5, SP6 and SP7, and a lack of detection of SP1 and SP3.

### B. Marker Set:2 Cell Lysate

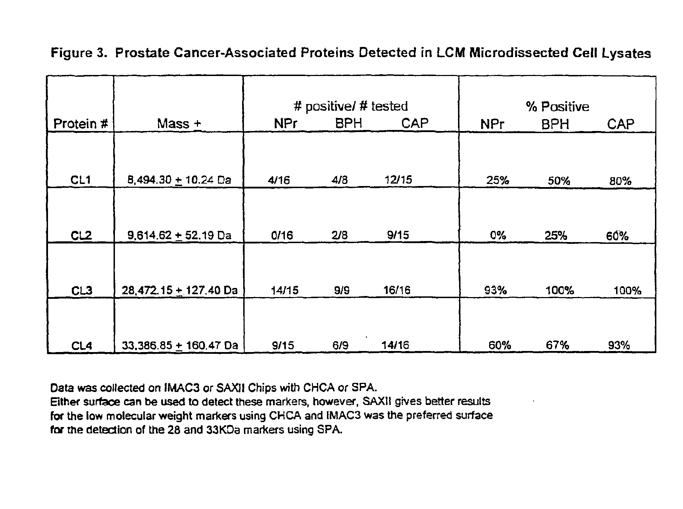

FIG 3 is a chart of the detection of markers CL1 to CL4 in CaP, BPH and negative diagnosis. Compared to a negative diagnosis, Marker CL1 and Marker CL2 are more frequently detected in both CaP and BPH. (CL2 is not detected in negative diagnosis.) Therefore, detection of either or both of these markers is positively correlated with a pathologic diagnosis.

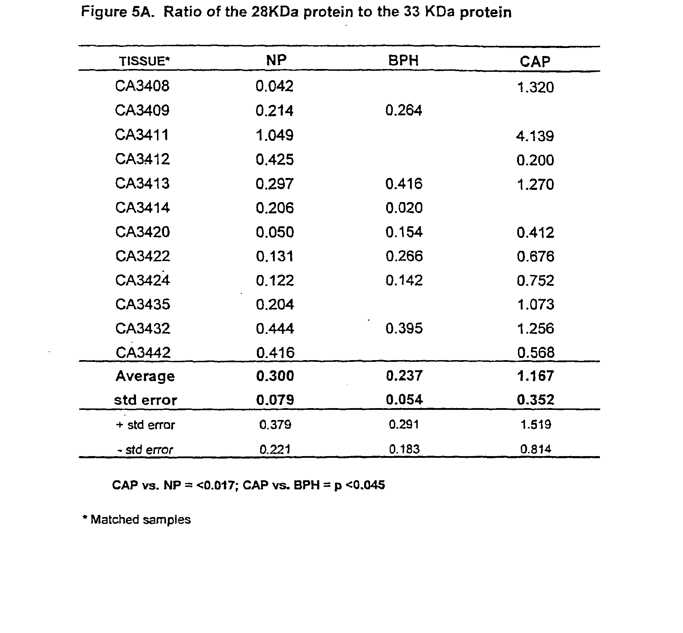

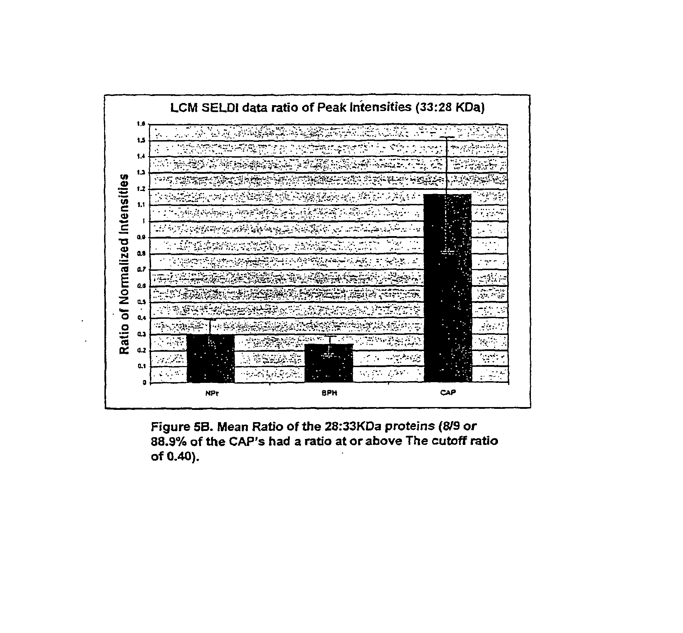

Markers CL3 and CL4 are detected in all three states. FIGS 5A and 5B show a chart and a graph of the ratio of CL3 and CL4 in various tissue samples tested, including the average ratio, which is presented in the bar graph of FIG 5B. The relative quantity can be determined in gas phase ion spectrometry either by peak height or peak area. Preferably, this determination is normalized with respect to an internal standard, *e.g*., a peak at 11400 D, which is present in all samples. As can be seen, a ratio of CL4:CL3 which is greater than 0.4 is positively correlated with CaP. The confidence of this diagnosis increases when the ratio is at least 0.75 and at least 1.0.

### C. Detection of Markers: Comparison with Controls

Detection of markers can usefully involve comparing the test data to a control. The control can be useful for calibration of "normal" amounts and for quantifying the amount of a marker against a normal or control amount. For example, it can be useful to know whether a marker present in both normal and pathologic conditions is increased by 1.5 fold, by 2 fold, by 5 fold, or by 10 fold compared to the control amount.

In one embodiment, the control amount can be an amount of a marker present in comparable samples from normal patients. The control amount is measured under the same or substantially similar experimental conditions as in measuring the test amount. For example, if a test sample is obtained from a subject's seminal plasma and a marker is detected using a particular probe, then a control amount of the marker is preferably determined from a seminal plasma sample of a patient using the same probe. It is preferred that the control amount of marker is determined based upon a significant number of samples from subjects who do not have prostate cancer (*e.g*., BPH or negative diagnosis patients) so that it reflects variations of the marker amounts in that population.

### D. Correlation By Computer

Data generated by mass spectrometry is usefully analyzed by computer software. The software can comprise code that converts signal from the mass spectrometer into computer readable form. The software also can include code that applies an algorithm to the analysis of the signal to determine whether the signal represents a "peak" in the signal corresponding to a marker of this invention, or other useful markers. The software also can include code that executes an algorithm that compares signal from a test sample to a typical signal characteristic of "normal," "BPH" and "CaP" and determines the closeness of fit between the two signals. The software also can include code indicating which the test sample is closest to, thereby providing a probable diagnosis.

### V. KITS

In yet another aspect, the invention provides kits for aiding a diagnosis of prostate cancer, wherein the kits can be used to detect the markers of the present invention. For example, the kits can be used to detect any one or combination of markers described above, which markers are differentially present in samples of a prostate cancer patient, BPH and normal patients. The kits of the invention have many applications. For example, the kits can be used to differentiate if a subject has prostate cancer, BPH or has a negative diagnosis, thus aiding a prostate cancer diagnosis. In another example, the kits can be used to identify compounds that modulate expression of the markers in *in vitro* prostate cells or *in vivo* animal models for prostate cancer.

In one embodiment, a kit comprises: (a) a substrate comprising an adsorbent thereon, wherein the adsorbent is suitable for binding a marker, and (b) a washing solution or instructions for making a washing solution, wherein the combination of the adsorbent and the washing solution allows detection of the marker using gas phase ion spectrometry. Such kits can be prepared from the materials described above, and the previous discussion of these materials (*e.g*., probe substrates, adsorbents, washing solutions, *etc*.) is fully applicable to this section and need not be repeated.

In some embodiments, the kit may comprise a first substrate comprising an adsorbent thereon (*e.g*., a particle functionalized with an adsorbent) and a second substrate onto which the first substrate can be positioned to form a probe which is removably insertable into a gas phase ion spectrometer. In other embodiments, the kit may comprise a single substrate which is in the form of a removably insertable probe with adsorbents on the substrate.

Optionally, the kit can further comprise instructions for suitable operational parameters in the form of a label or a separate insert. For example, the kit may have standard instructions informing a consumer how to wash the probe after a sample of seminal plasma is contacted on the probe.

In another embodiment, a kit comprises (a) an antibody that specifically binds to a marker; and (b) a detection reagent. Such kits can be prepared from the materials described above, and the previous discussion regarding the materials (e.g., antibodies, detection reagents, immobilized supports, *etc.*) is fully applicable to this section and need not be repeated.

In either embodiment, the kit may optionally further comprise a standard or control information so that the test sample can be compared with the control information standard to determine if the test amount of a marker detected in a sample is a diagnostic amount consistent with a diagnosis of prostate cancer.

### EXAMPLES

The following examples show the identification of two sets of markers that have positive or negative correlations with prostate cancer or benign prostate hyperplasia. A first set was determined from samples of seminal plasma. A second set of markers was determined from samples of cell lysate isolated by laser capture microdissection.

### I. TISSUE AND SEMEN SPECIMENS

Tissue and semen specimens were obtained from the Virginia Prostate Center tissue and body fluid bank. Prostate tissue specimens were procured at the time of surgery, immediately frozen in liquid nitrogen, and stored at -80°C. Semen was collected from patients seen in the Department of Urology after informed consent. The semen samples were processed as previously described and stored at -80°C. The semen samples were either processed immediately after collection or stored frozen at -80° until processed. For processing, the semen samples were mixed with one-half volume of dilution buffer (pH 8.0) consisting of 123 mM NaCl, 5 m KCl, 1 mM MgSO₄, 37 mM Tris, 1 mM EDTA and 2.5 mM of a serine protease inhibitor cocktail (PMSF or PEFABLOC). The samples were then vortexed to ensure complete mixing, centrifuged at 25,000 xG for 5 minutes, and the supernatant distributed in 100 µl to 500 µl aliquots and store at -80° C.

### II. LASER CAPTURE MICRODISSECTION

Populations of normal and prostate cancer cells were procured from frozen tissue sections using the PixCell^{™} Laser Capture Microdissection Microscope (Arcturus Engineering, Inc., Mountain View, CA) essentially as described by Emmert-Buck et. al., "Laser capture microdissection," *Science* 1996; **274**: 998-1001. See also United States Patent 5,843,644 (Liotta et al.) and United States Patent 5,843,657 (Liotta et al.).

### III. LASER DESORPTION/IONIZATION MASS SPECTROMETRY

Samples were analyzed by laser desorption/ionization mass spectrometry. The system used was a PBSI or PBSII obtained from Ciphergen Biosystems, Inc. (Palo Alto, CA). These systems employ a nitrogen laser at 337.1 nm. The pulse width is 4 nanoseconds. The maximum energy output of the systems is about 150 micro Joules per pulse. However, in these studies, photo energy of about 1-25 µJ per pulse was used.

### IV. MARKERS IN SEMINAL PLASMA

### A. Protocol for Processing Seminal Plasma

### 1. Serum and Seminal Plasma

Serum samples were diluted (20µl in 30µl 8 M urea, 1% CHAPS, in PBS) and depleted of albumin by using a cibracon blue spin column. Seminal plasma (SP) samples were diluted (20µl SP with 30µl of a buffer containing 9.5M urea, 2% CHAPS, 50 mM Tris pH 9.5) followed by a further dilution in 1:9 in 50 mM HEPES. For each sample type, 5µl was spotted onto SAX, Cu-IMAC or Mixed Mode (anionic and hydrophobic surfaces) ProteinChip™ arrays, and mass analysis performed using sinapinic acid as the matrix.

### 2. Preparation of Seminal Plasma:

1. Seminal plasma samples were collected, cells were spun out and protease inhibitors were added. Samples were aliquoted and stored at -70°C.
2. Protein concentrations were determined by BCA method and standardized for each sample by diluting to a concentration of 23 mg/ml in binding buffer (see below).
3. After quick spinning the samples at 14K for 2 min., supernatant were diluted in a binding buffer containing 20 mM Tris pH 9.0, 0.4M NaCl, and 0.1% Triton X100 in a volume of 100 microliters. (90µl concentrated adjusted seminal plasma, 10µl 5M NaCl, 1µl 10% Tx100) The samples were then left on ice for 30 minutes.

### 3. Preparation of Size Selection Columns and Size Fractionation:

1. Break the outlet cap of the K30 Spin column. Insert the column into a 2ml screw-cap tube.
2. Open the top cap of the Spin column. Spin the column at 3000 rpm (720g) for a 3 minutes at room temperature. The column matrix should be packed down and semi-dry, but not cracked.
3. Transfer the spin column to a new 2ml tube. Apply 30µl of the diluted seminal plasma slowly to the center of the column matrix. Do not allow the samples to run down the side of the matrix.
4. Centrifuge columns at 3000 rpm for 3 minutes. The fractionated proteins are in the collection tube. This is fraction 1.
5. Transfer the spin column to a new tube. Add 30µl of binding buffer (20 mM TRIS pH 9, .4M NaCl, 0.1%Tx) to column and spin as above. This is Fraction 2.
6. Repeat step 5 and collect two more fractions.
7. Total of 4 fractions are collected. Store fractions on ice.

### 4. Preparation of Anion Exchange Column for Protein Fractionation:

1. Combine fractions 1 & 2 and fractions 3 & 4 from the size selection above. The total volume was adjusted to 120µl with binding buffer (i.e. you will have approx. 50-60µl volume total, add 60-70µl binding buffer). This is labeled UN (Un-fractionated).
2. Break the outlet cap of the anion exchange Q column. Insert a 2ml tube. Open the top cap.
3. Centrifuge the column at 1000 rpm (80g) for 1 minute. The column matrix should be packed, but not cracked.
4. Transfer the column to a new 2 ml tube. Apply 90µl of the combined fraction samples (you will have a total of 2, F1&2 and F3&4). Apply the sample slowly to the center of the matrix. Incubated at room temperature for 5 min.
5. Centrifuge the column(s) at 1000 rpm for 1 minute. The proteins in the collection column are the flow through fraction. These proteins do not bind to the column because they have a neutral or positive net charge in the binding buffer, or they may be coming through because the capacity of the resin is met.
6. To maximize the capture of proteins, the eluent is re-applied to the column, incubated for 3 min at room temperature, centrifuge as above. This flow through is fraction Q1.
7. Transfer the column to a new tube. Wash the column with 100µl of binding buffer, incubated at room temperature for 3 min, centrifuge as above. Repeat this step. Collect total 200µl for this fraction Q2.
8. Transfer the column to a third tube. Apply 100µl of the elution Buffer A (pH 8.0), incubate at room temperature for 3 min., centrifuge as above. This is fraction Q3.
9. Continue this process for subsequent fractions with the elution buffers (Buffers B-E, pH 7-4).
10. For each sample, 16 fractions are collected after size and anionic columns. UN(1+2), nl(1+2), Q2(1+2), Q4(1+2), Q5(1+2), Q6(1+2), Q7(1+2), UN(3+4), Q1(3+4), Q2(3+4), Q3(3+4), Q4(3+4), Q5(3+4), Q6(3+4), Q7(3+4).

### 5. SELDI processing of Seminal Plasma Fractions:

1. Spot 2µl of each fraction on a Normal Phase Chip. Draw a hydrophobic ring around each array before spotting. Let it dry completely before spot 0.5µl SPA matrix twice using 50% acetonitrile with.5% TFA, and 0.6 % Triton X as the solvent.
2. Read the Chips on laser intensity 15, sensitivity of 10, with the filter in for a low mass reading and at laser intensity 50 with the filter in for high mass collection. Collect 70 shots for the combined average spectra (10 shots over 7 regions, i.e., 20-80).

### 6. Results:

View the spectra for each reading and use a filter (50 and 150 is adequate for L- 15 and L50, respectively). Use the auto-peak identification mode with settings of 5 times the noise.

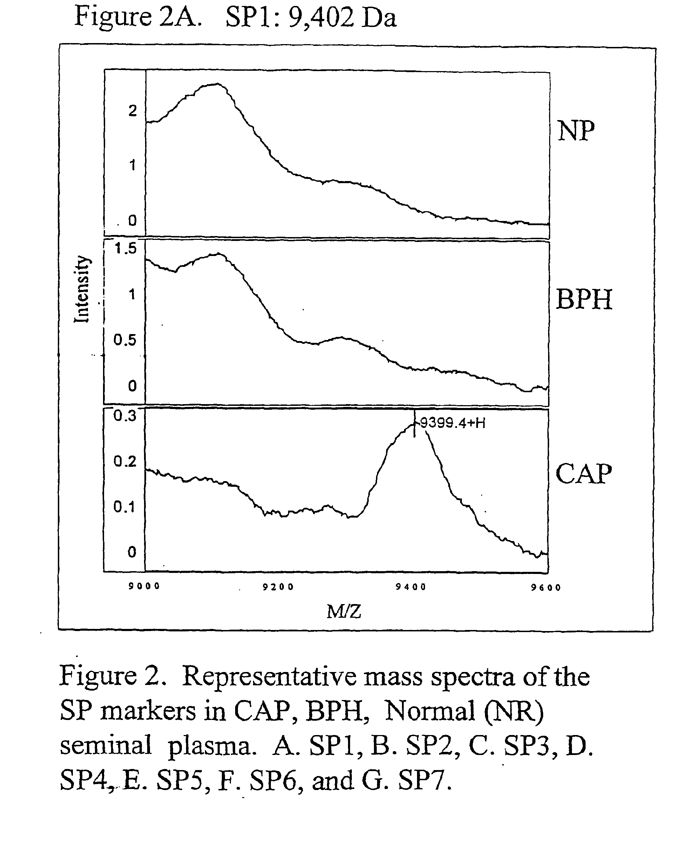

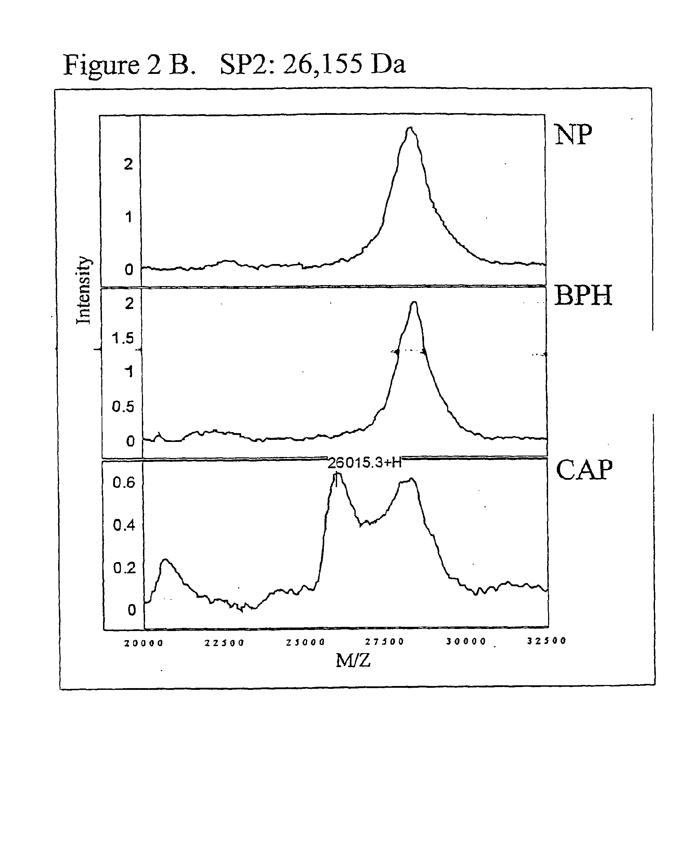

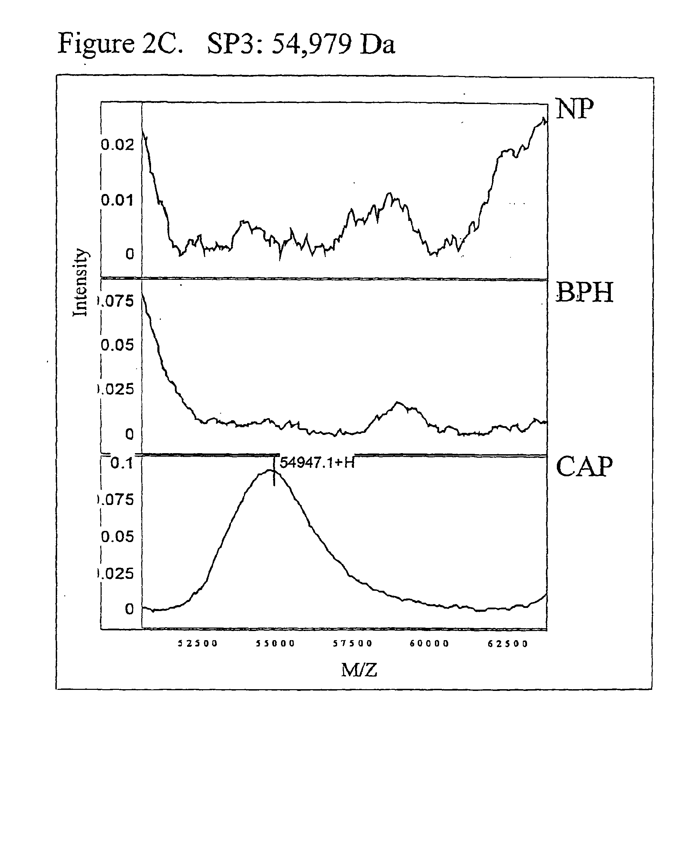

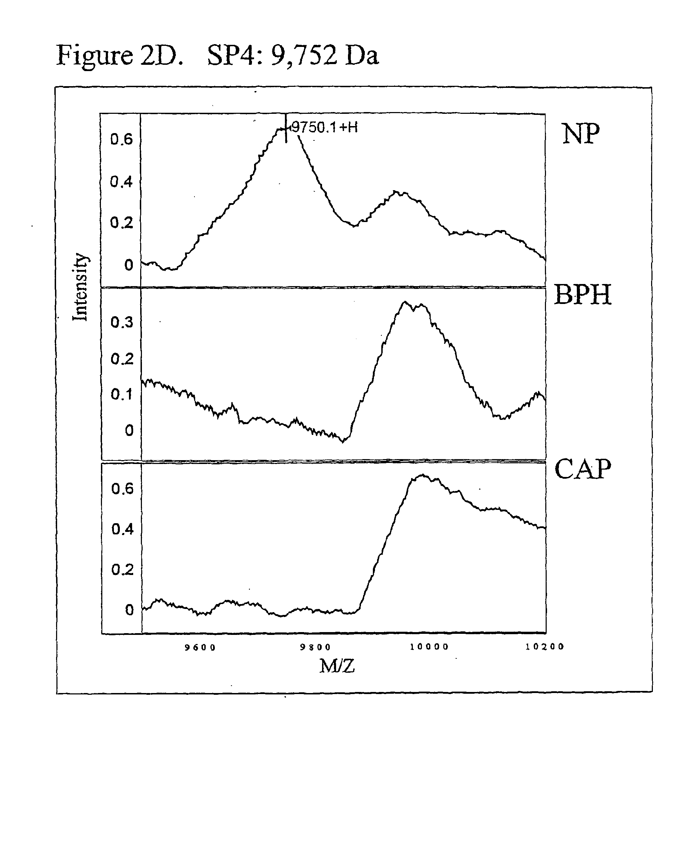

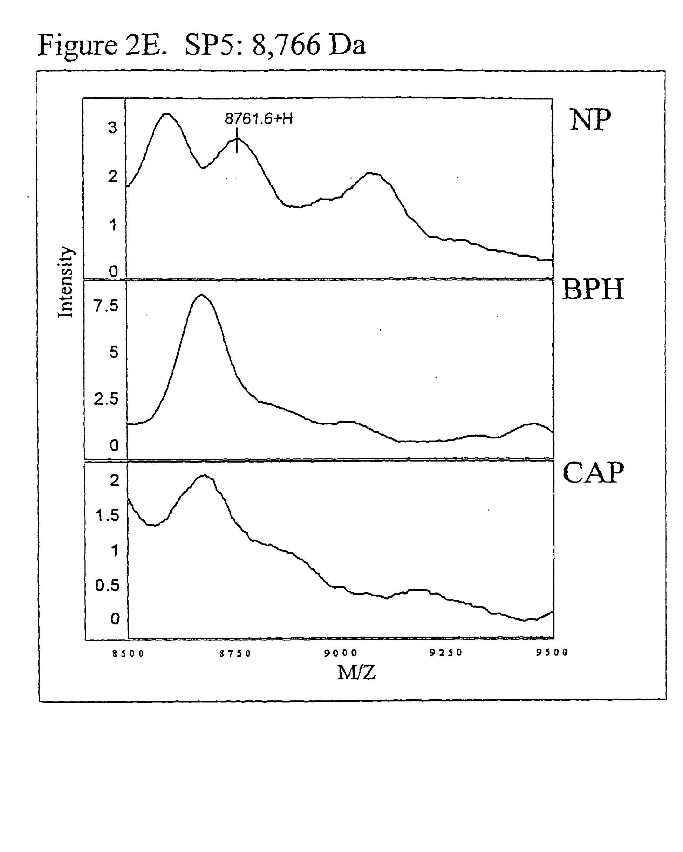

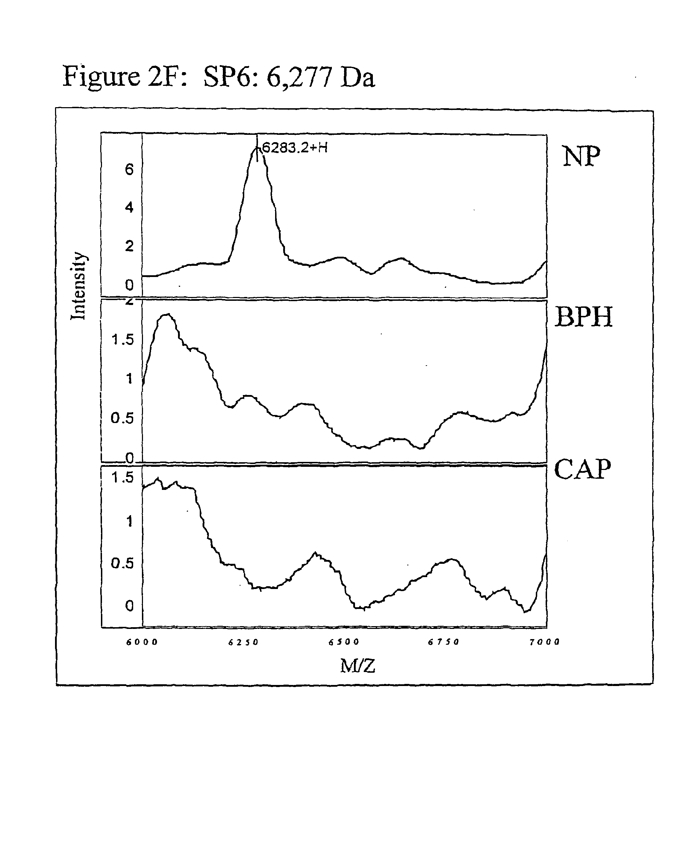

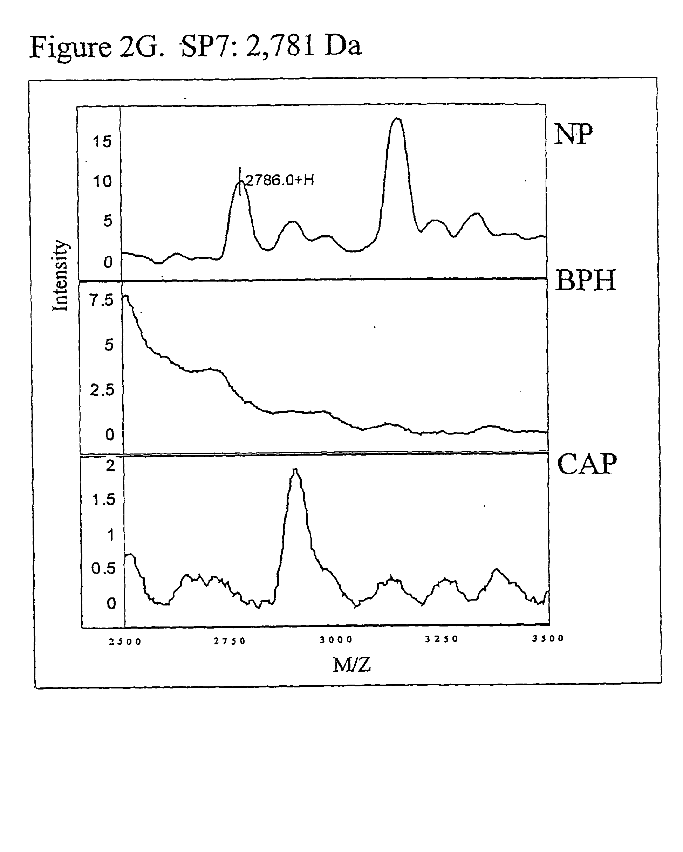

Data was collected from nine CAP (carcinoma of prostate), nine BPH (Benign prostate hyperplasia), and nine NR (normal prostate). Data was analyzed one fraction at a time. Seven potential biomarkers were observed. FIGS 2A-2G present typical traces from negative, BPH and CaP samples showing the following markers: Marker SP1: 9,402.68 ± 8.97 Da, Marker SP2: 26,155.30 ± 202.01 Da, Marker SP3: 54,979.27 ± 408.78 Da, Marker SP4: 9,752.30 ± 15.08 Da, Marker SP5: 87,66.93 ± 14.44 Da, Marker SP6: 62,77.97 ± 12.36 Da and Marker SP7: 2,781.72 ± 4.41 Da.

### V. MARKERS IN CELL LYSATES FROM PROSTATE EPITHELIAL TISSUE

Markers from prostate epithelial tissue cell lysates were identified by SELDI using a metal chelate adsorbent or using a hydrophilic adsorbent. A total of 2000-3000 microdissected cells were lysed in 5-10 ul of 5M guanidinium isothiocyanate in buffer, or 20 mM HEPES with 0.1 % Triton X-100. The entire lysate was then spotted on LMAC3 (nickel metal chelate) or SAX2 (Strong Anionic Exchanger: quaternary ammonium) ProteinChip^{™} arrays with washing. Mass analysis was performed in the SELDI PBS I using α-cyano-4-hydroxy cinnamic acid for the energy absorbing molecule. Chips and mass spectrometer were obtained from Ciphergen Biosystems, Inc. (Palo Alto, California).

### A. IMAC3 ProteinChip

### 1. Protocol:

1. Draw a hydrophobic ring around each adsorbent spot on the chip.
2. Load 10µl 50 mM NiSO₄. Incubate chip for 5 minutes in a moist chamber. With shaking. Repeat.
3. Wash Chip with DI H₂0 10 seconds, around spots and remove excess water.
4. Load 5µl of binding buffer (20 mM Tris pH 7.5, 0.1% TritonX 100, 0.5M NaCl)
   Incubate chip for 5 minutes in a moist chamber. With shaking.
5. Remove binding buffer carefully with a Kim wipe.
6. Lyse microdissected cells (500-1000 shots) in 5µl of 20 mM HEPES + 0.1% NP 40 directly on the cap. Pipette the lysis buffer up and down on top of the cells until lysis occurs. Vortex cell lysate 1 minute and spin down.
7. Spot the entire lysate (5µl) on spot and incubate in a moist chamber 30 minutes to one hour.
8. Wash each spot 5x with 5µl of binding buffer or in bulk.
9. Wash 2x 5 µl with water.
10. Spot 2x 0.5µl SPA or CHCA in 50% ACN, .5% TFA with 0.06% Tx while spots are still moist.
11. Air dry.

### 2. SELDI Reader Data Collection

1. Peaks were collected on the Ciphergen Biosystems PBSI at a laser intensity of 15, 30 and 50 (no filter) and for PBSII a laser intensity of 230 and 260 with a detector sensitivity of 10. 70 shots were collected and averaged.
2. Normalization of peak intensities was performed using an internal
   standard: i.e. a peak present at or about same level in all specimens tested. **Notes:**
   The LCM samples averaged 1000 shots, which is equivalent to 2000-3000 cells.

### B. SAX-1 ProteinChip

### 1. Protocol:

1. Draw a hydrophobic ring around each adsorbent spot on the array.
2. Load 5µl 20 mM Tris pH 7.5 + .1% Triton X100 (binding buffer).
   Incubate chip for 30 minutes in a moist chamber. Repeat one more time.
3. Lyse microdissected cells in 5 µl of 5M GITC buffer directly on the cap.
   Pipette the lysis buffer up and down on top of the cells until lysis occurs.
4. Dilute lysates 1:10 in binding buffer in a total of 10 µl. Spot 4 µl of lysate and incubate in a moist chamber 30 minutes to one hour. Remove excess samples and repeat with another 4µl of lysate.
5. Wash each spot 5x with 5µl of binding buffer (20 mM TRIS pH 7.5, 0.1%
   TX100). Wash 2x 5µl with water.
6. Spot 2x 0.5µl CHCA in 50% ACN, .5% TFA while spots are still moist.
7. Air dry.

### 2. SELDI Reader Data Collection and Results

1. Peaks were collected at a laser intensity of 10 and 15 on the PBSI with the filter in and a detector sensitivity of 10. 60 shots were collected.

### C. Results

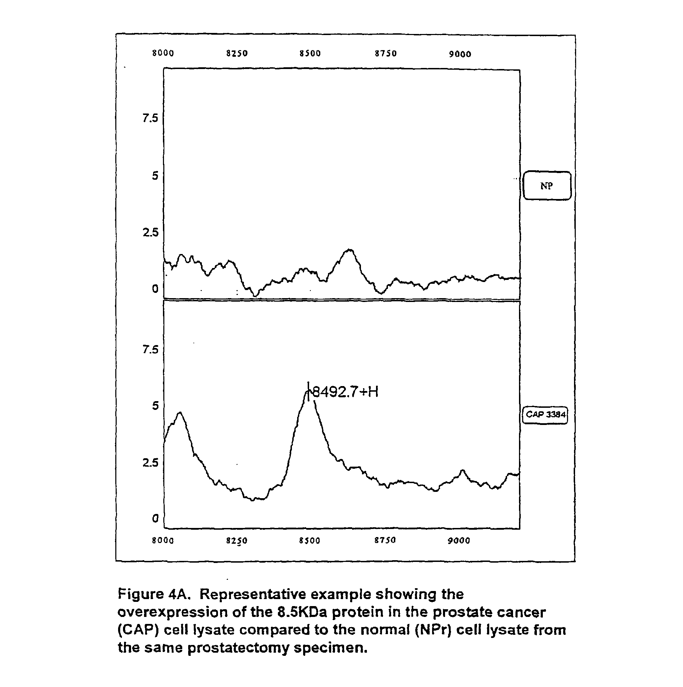

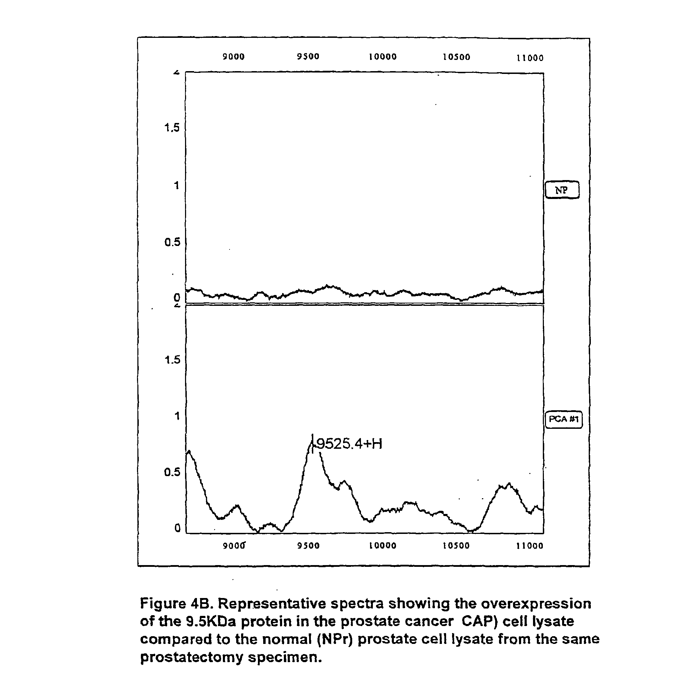

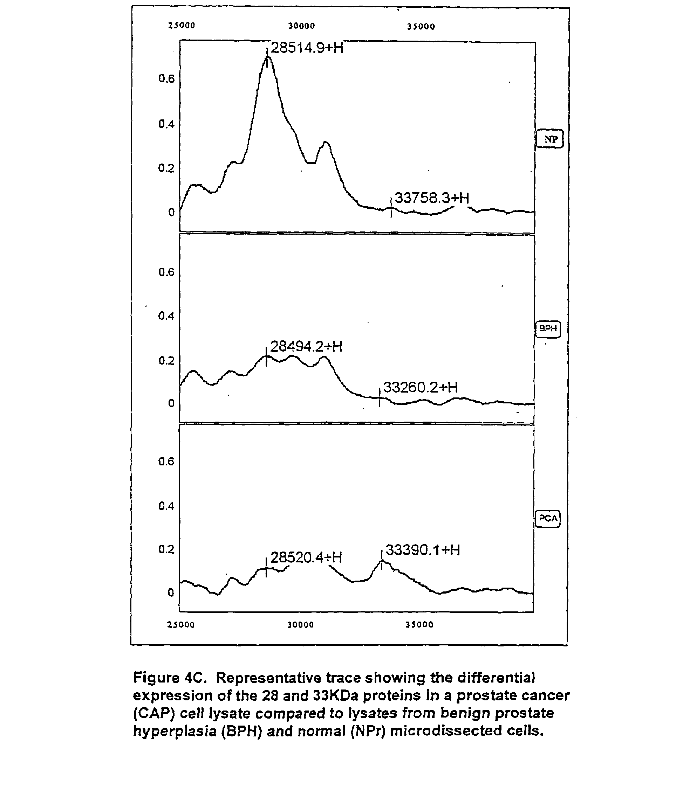

FIGS 4A-4C present typical traces in negative, BPH and CaP samples depicting the following useful markers: Marker CL1: 8,494.30 ± 10.24 Da, Marker CL2: 9,614.62 ± 52.19 Da, Marker CL3: 28,472 ± 127.40 Da, and Marker CL4: 33,386.85 ± 160.47 Da. FIG 5A presents a chart showing the relative amounts of CL4:CL3 in various samples. FIG 5B is a bar graph showing these ratios with statistical error bars.

## Claims

1. A method for aiding in a differential diagnosis of prostate cancer, benign prostate hyperplasia, and a negative diagnosis, comprising:
(a) detecting at least one protein marker in a first sample from a subject, wherein the protein marker is selected from:
Marker SP1: 9,402.68 ± 8.97 Da, and
Marker SP3: 54,979.27 ± 408.78 Da; and
(b) correlating the detection of the marker or markers from step (a) with a probable diagnosis of prostate cancer, benign prostate hyperplasia or a negative diagnosis, wherein the correlation takes into account the relative detectability of the marker or markers in each diagnosis.

2. A method according to Claim 1, further comprising detecting at least one protein marker in said first sample selected from:
Marker SP2: 26,155.30 ± 202.01 Da,
Marker SP5: 8,766.93 ± 14.44 Da,
Marker SP6: 6,277.97 ± 12.36 Da, and
Marker SP7: 2,781.72 ± 4.41 Da.

3. A method according to Claim 1 or Claim 2, further comprising detecting at least one protein marker in a second sample from said subject, wherein said protein marker is selected from:
Marker CL1: 8,494.30 ± 10.24 Da, and
Marker CL2: 9,614.62 ± 52.19 Da.

4. A method according to any of Claims 1-3, further comprising measuring the amount of Marker CL3: 28,472 ± 127.40 Da and Marker CL4: 33,386.85 ± 160.47 Da in a second sample from said subject, and determining the ratio of the amounts of Marker CL4 to Marker CL3, and correlating the ratio with a probable diagnosis of prostate cancer, benign prostate hyperplasia or a negative diagnosis, wherein the correlation takes into account the relative detectability of the markers in the diagnosis.

5. A method according to any previous claim, comprising detecting the marker or markers by gas phase ion spectrometry.

6. A method according to Claim 5, wherein said gas phase ion spectrometry is laser desorption mass spectrometry.

7. A method according to any of Claims 1-4, comprising detecting the marker or markers by immunoassay.

8. A method according to any of Claims 1-4, wherein said first sample is seminal plasma.

9. A method according to any of Claims 1-4, wherein said first sample is selected from the group consisting of blood, serum, urine, prostatic fluid, seminal fluid, semen, and prostate tissue.

10. A method according to any previous claim, comprising detecting Marker SP2 and Marker SP1.

11. A method according to any of Claims 1-9, comprising detecting Marker SP2.

12. A method according to Claim 11, further comprising detecting at least either or both of Marker SP1 and a Marker selected from SP4: 9,752.30 ± 15.08 Da, SP5, SP6 and SP7.

13. A method according to Claim 12, comprising detecting Marker SP2, Marker SP1, and a Marker selected from the group consisting of SP4, SP5, SP6 and SP7.

14. A method according to Claim 13, comprising detecting Marker SP4.

15. A method according to Claim 13, comprising detecting Marker SP5.

16. A method according to Claim 13, comprising detecting Marker SP6.

17. A method according to Claim 13, comprising detecting Marker SP7.

18. A method according to any of Claims 3-17, wherein said second sample is prostate tissue extract.

19. A method according to any of Claims 3-17, wherein said second sample is selected from the group consisting of blood, serum, urine, prostatic fluid, seminal fluid, semen, seminal plasma, and prostate tissue.

20. A method according to any previous claim, comprising detecting Marker CL2: 9,614.62 ± 52.19 Da.

21. A method according to any previous claim, comprising detecting Marker CL1: 8,494.30 ± 10.24 Da.

22. A method according to any previous claim, comprising detecting Marker CL2: 9,614.62 ± 52.19 Da and CL1: 8,494.30 ± 10.24 Da.

23. A method according to Claim 6, comprising:
(i) generating data on the sample with the mass spectrometer indicating intensity of signal for mass/charge ratio,
(ii) transforming the data into computer-readable form;
(iii) executing an algorithm with a programmable digital computer, wherein the algorithm determines closeness-of-fit between the computer-readable data and data indicating a diagnosis of CaP, BPH or a negative diagnosis.

24. A method according to Claim 6, comprising:
(i) generating data on the sample with the mass spectrometer indicating intensity of signal for mass/charge ratio,
(ii) transforming the data into computer-readable form; and
(iii) executing an algorithm with a programmable digital computer wherein the algorithm detects signal in the computer-readable data representing the marker or markers.

25. A method according to Claim 6, wherein laser desorption/ionization mass spectrometry comprises:
(i) providing a probe adapted for use with a mass spectrometer comprising an adsorbent attached thereto;
(ii) contacting the marker or markers with the adsorbent; and
(iii) desorbing and ionizing the marker or markers from the probe and detecting the desorbed/ionized marker or markers with the mass spectrometer.

26. A method according to Claim 6, wherein laser desorption/ionisation mass spectrometry comprises:
(i) providing a substrate comprising an adsorbent attached thereto;
(ii) contacting the marker or markers with the adsorbent;
(iii) placing the substrate on a probe adapted for use with a mass spectrometer comprising an adsorbent attached thereto; and
(iv) desorbing and ionizing the marker or markers from the probe and detecting the desorbed/ionized marker or markers with the mass spectrometer.

27. A method according to Claim 25, wherein the adsorbent is a hydrophilic adsorbent or a metal chelate adsorbent.

28. A method according to Claim 27, wherein the adsorbent is a hydrophilic adsorbent comprising silicon oxide.

29. A method according to Claim 27, wherein the adsorbent is a metal chelate adsorbent comprising nickel.

30. A method according to Claim 25, wherein the adsorbent comprises an antibody that specifically binds to the marker.

31. A method according to Claim 5, further comprising, before detecting the marker or markers, fractionating the sample by size exclusion chromatography and collecting a fraction that includes the marker or markers.

32. A method according to Claim 5, wherein the method further comprises, before detecting the marker or markers, fractionating the sample by anion exchange chromatography and collecting a fraction that includes the marker or markers.

33. A method according to Claim 5, wherein when Markers CL1, CL2, CL3 or CL4 are detected, the method further comprises,
(i) before detecting the marker or markers, fractionating a sample comprising the marker or markers by contacting the sample with a substrate comprising an adsorbent that retains the marker or markers and removing unretained sample; and
(ii) desorbing and ionizing the retained markers form the adsorbent during mass spectrometry.

34. A method according to Claim 33, wherein the substrate is a mass spectrometer probe comprising the adsorbent on a probe surface.

35. A method according to Claim 34, wherein the substrate is a resin, and after fractionating the sample, the resin with the marker or markers retained by the adsorbent is placed on a mass spectrometer probe for desorption and ionization by the mass spectrometer.

36. A method according to Claim 33, wherein the adsorbent is selected from a hydrophilic adsorbent and a metal chelate adsorbent.

37. A method according to Claim 33, wherein the adsorbent is a strong anion exchange adsorbent or a nickel chelate adsorbent.

38. A kit for aiding in the differential diagnosis of prostate cancer, benign prostate hyperplasia, and a negative diagnosis, comprising:
(1) an adsorbent attached to a substrate, wherein the adsorbent retains at least one protein marker selected from:
Marker SP1: 9,402.68 ± 8.97 Da, and
Marker SP3: 54,979.27 ± 408.78 Da; and
(2) instructions to detect the marker or markers by contacting a sample with the adsorbent and detecting the marker or markers retained by the adsorbent,
wherein said adsorbent comprises at least one antibody selected from an antibody that specifically binds Marker SP1 and an antibody that specifically binds Marker SP3.

39. A kit according to Claim 38, wherein said adsorbent retains at least one further protein marker selected from:
Marker SP2: 26,155.30 ± 202.01 Da,
Marker SP5: 8,766.93 ± 14.44 Da,
Marker SP6: 6,277.97 ± 12.36 Da, and
Marker SP7: 2,781.72 ± 4.41 Da.

40. A kit according to Claim 38 or 39, wherein said adsorbent retains at least one further protein marker selected from:
Marker CL1: 8,494.30 ± 10.24 Da, and
Marker CL2: 9,614.62 ± 52.19 Da.

41. A kit according to any of Claims 38-40, wherein said adsorbent retains at least one further protein marker selected from:
Marker CL3: 28,472 ± 127.40 Da, and
Marker CL4: 33,386.85 ± 160.47 Da.

42. A kit according to any of Claims 38-41, wherein the substrate is a probe for a gas phase ion spectrometer having a surface on which the adsorbent is attached.

43. A kit according to any of Claims 38-41, further comprising
(i) an eluent wherein the marker or markers are retained on the adsorbent when washed with the eluent, or
(ii) instructions to wash adsorbent with the eluent after contacting the adsorbent with the marker or markers.

44. A kit according to any of Claims 38-43, wherein the adsorbent further comprises a hydrophilic adsorbent or a metal chelate adsorbent.

45. A kit according to Claim 44, wherein the hydrophilic adsorbent further comprises silicon oxide.

46. A kit according to any of Claims 38-43, wherein the adsorbent further comprises an anionic exchange adsorbent.

47. A kit according to any of Claims 38-43, wherein the adsorbent further comprises a nickel metal chelate adsorbent.

## Patentansprüche

1. Verfahren zur Unterstützung bei einer Differentialdiagnose von Prostatakrebs, benigner Prostatahyperplasie und einer negativen Diagnose, umfassend:
(a) Nachweisen wenigstens eines Proteinmarkers in einer ersten Probe von einem Subjekt, wobei der Proteinmarker ausgewählt ist aus:
Marker SP1: 9.402,68 ± 8,97 Da und
Marker SP3: 54.979,27 ± 408,78 Da; und
(b) Korrelieren des Nachweises des Markers oder der Marker von Schritt (a) mit einer wahrscheinlichen Diagnose von Prostatakrebs, benigner Prostatahyperplasie oder einer negativen Diagnose, wobei die Korrelation die relative Nachweisbarkeit des Markers oder der Marker bei jeder Diagnose berücksichtigt.

2. Verfahren nach Anspruch 1, ferner umfassend Nachweisen wenigstens eines Proteinmarkers in der ersten Probe, ausgewählt aus:
Marker SP2: 26.155,30 ± 202,01 Da,
Marker SP5: 8.766,93 ± 14,44 Da,
Marker SP6: 6.277,97 ± 12,36 Da und
Marker SP7: 2.781,72 ± 4,41 Da.

3. Verfahren nach Anspruch 1 oder Anspruch 2, ferner umfassend Nachweisen wenigstens eines Proteinmarkers in einer zweiten Probe von dem Subjekt, wobei der Proteinmarker ausgewählt ist aus:
Marker CL1: 8.494,30 ± 10,24 Da und
Marker CL2: 9.614,62 ± 52,19 Da.

4. Verfahren nach irgendeinem der Ansprüche 1-3, ferner umfassend Messen der Menge an Marker CL3: 28.472 ± 127,40 Da und Marker CL4: 33.386,85 ± 160,47 Da in einer zweiten Probe von dem Subjekt, und Bestimmen des Verhältnisses der Mengen an Marker CL4 zu Marker CL3 und Korrelieren des Verhältnisses mit einer wahrscheinlichen Diagnose von Prostatakrebs, benigner Prostatahyperplasie oder einer negativen Diagnose, wobei die Korrelation die relative Nachweisbarkeit der Marker bei der Diagnose berücksichtigt.

5. Verfahren nach irgendeinem vorhergehenden Anspruch, umfassend Nachweisen des Markers oder der Marker durch Gasphasenionenspektrometrie.

6. Verfahren nach Anspruch 5, wobei die Gasphasenionenspektrometrie Laserdesorptionsmassenspektrometrie ist.

7. Verfahren nach irgendeinem der Ansprüche 1-4, umfassend Nachweisen des Markers oder der Marker durch Immunoassay.

8. Verfahren nach irgendeinem der Ansprüche 1-4, wobei die erste Probe Samenplasma ist.

9. Verfahren nach irgendeinem der Ansprüche 1-4, wobei die erste Probe ausgewählt ist aus der Gruppe bestehend aus Blut, Serum, Urin, Prostataflüssigkeit, Samenflüssigkeit, Samen und Prostatagewebe.

10. Verfahren nach irgendeinem vorhergehenden Anspruch, umfassend Nachweisen von Marker SP2 und Marker SP1.

11. Verfahren nach irgendeinem der Ansprüche 1-9, umfassend Nachweisen von Marker SP2.

12. Verfahren nach Anspruch 11, ferner umfassend Nachweisen wenigstens eines oder beides von Marker SP1 und einem Marker, ausgewählt aus SP4: 9.752,30 ± 15,08 Da, SP5, SP6 und SP7.

13. Verfahren nach Anspruch 12, umfassend Nachweisen von Marker SP2, Marker SP1 und einem Marker, ausgewählt aus der Gruppe bestehend aus SP4, SP5, SP6 und SP7.

14. Verfahren nach Anspruch 13, umfassend den Nachweis von Marker SP4.

15. Verfahren nach Anspruch 13, umfassend Nachweisen von Marker SP5.

16. Verfahren nach Anspruch 13, umfassend Nachweisen von Marker SP6.

17. Verfahren nach Anspruch 13, umfassend Nachweisen von Marker SP7.

18. Verfahren nach irgendeinem der Ansprüche 3-17, wobei die zweite Probe Prostatagewebeextrakt ist.

19. Verfahren nach irgendeinem der Ansprüche 3-17, wobei die zweite Probe ausgewählt ist aus der Gruppe bestehend aus Blut, Serum, Urin, Prostataflüssigkeit, Samenflüssigkeit, Samen, Samenplasma und Prostatagewebe.

20. Verfahren nach irgendeinem vorhergehenden Anspruch, umfassend Nachweisen von Marker CL2: 9.614,62 ± 52,19 Da.

21. Verfahren nach irgendeinem vorhergehenden Anspruch, umfassend Nachweisen von Marker CL1: 8.494,30 ± 10,24 Da.

22. Verfahren nach irgendeinem vorhergehenden Anspruch, umfassend Nachweisen von Marker CL2: 9.614,62 ± 52,19 Da und CL1: 8.494,30 ± 10,24 Da.

23. Verfahren nach Anspruch 6, umfassend:
(i) Generieren von Daten an der Probe mit dem Massenspektrometer, die die Signalintensität für das Verhältnis von Masse/Ladung anzeigen;
(ii) Transformieren der Daten in computerlesbare Form;
(iii) Ausführen eines Algorithmus mit einem programmierbaren Digitalcomputer, wobei der Algorithmus "closeness-of-fit" zwischen den computerlesbaren Daten und Daten, die eine Diagnose von CaP, BPH oder eine negative Diagnose anzeigen, bestimmt.

24. Verfahren nach Anspruch 6, umfassend:
(i) Generieren von Daten an der Probe mit dem Massenspektrometer, die die Signalintensität für das Verhältnis von Masse/Ladung anzeigen;
(ii) Transformieren der Daten in computerlesbare Form; und
(iii) Ausführen eines Algorithmus mit einem programmierbaren Digitalcomputer, wobei der Algorithmus Signale in den computerlesbaren Daten auffindet, die den Marker oder die Marker darstellen.

25. Verfahren nach Anspruch 6, wobei die Laserdesorptions-/ionisationsmassenspektrometrie umfasst:
(i) Bereitstellen einer zur Verwendung mit einem Massenspektrometer geeigneten Sonde, die ein daran gebundenes Adsorptionsmittel umfasst;
(ii) In-Kontakt-Bringen des Markers oder der Marker mit dem Adsorptionsmittel; und
(iii) Desorbieren und Ionisieren des Markers oder der Marker von der Sonde und Nachweisen des desorbierten/ionisierten Markers oder der desorbierten/ionisierten Marker mit dem Massenspektrometer.

26. Verfahren nach Anspruch 6, wobei die Laserdesorptions-/ionisationsmassenspektrometrie umfasst:
(i) Bereitstellen eines Substrats, das ein daran gebundenes Adsorptionsmittel umfasst;
(ii) In-Kontakt-Bringen des Markers oder der Marker mit dem Adsorptionsmittel;
(iii) Aufbringen des Substrats, das ein daran gebundenes Adsorptionsmittel umfasst, auf eine zur Verwendung mit einem Massenspektrometer geeigneten Sonde, und
(iv) Desorbieren und Ionisieren des Markers oder der Marker von der Sonde und Nachweisen des desorbierten/ionisierten Markers oder der desorbierten/ionisierten Marker mit dem Massenspektrometer.

27. Verfahren nach Anspruch 25, wobei das Adsorptionsmittel ein hydrophiles Adsorptionsmittel oder ein Metallchelat-Adsorptionsmittel ist.

28. Verfahren nach Anspruch 27, wobei das Adsorptionsmittel ein hydrophiles Adsorptionsmittel ist, das Siliciumoxid umfasst.

29. Verfahren nach Anspruch 27, wobei das Adsorptionsmittel ein Nickel umfassendes Metallchelat-Adsorptionsmittel ist.

30. Verfahren nach Anspruch 25, wobei das Adsorptionsmittel einen Antikörper umfasst, der spezifisch an den Marker bindet.

31. Verfahren nach Anspruch 5, ferner umfassend, vor dem Nachweisen des Markers oder der Marker, Fraktionieren der Probe durch Größenausschlusschromatografie und Auffangen einer Fraktion, die den Marker oder die Marker enthält.

32. Verfahren nach Anspruch 5, wobei das Verfahren ferner umfasst, vor dem Nachweis des Markers oder der Marker, Fraktionieren der Probe durch Anionenaustauschchromatografie und Auffangen einer Fraktion, die den Marker oder die Marker enthält.

33. Verfahren nach Anspruch 5, wobei das Verfahren, wenn Marker CL1, CL2, CL3 oder CL4 nachgewiesen werden, ferner umfasst:
(i) vor dem Nachweisen des Markers oder der Marker, Fraktionieren einer Probe, die den Marker oder die Marker umfasst, indem man die Probe mit einem Substrat in Kontakt bringt, das ein Adsorptionsmittel umfasst, das den Marker oder die Marker zurückhält, und nicht zurückgehaltene Probe entfernt; und
(ii) Desorbieren und Ionisieren der zurückgehaltenen Marker von dem Adsorptionsmittel bei der Massenspektrometrie.

34. Verfahren nach Anspruch 33, wobei das Substrat eine Massenspektrometersonde ist, die das Adsorptionsmittel auf einer Sondenoberfläche umfasst.

35. Verfahren nach Anspruch 34, wobei das Substrat ein Harz ist, und das Harz nach Fraktionieren der Probe mit dem von dem Adsorptionsmittel zurückgehalten Marker oder mit den von dem Adsorptionsmittel zurückgehalten Markern zur Desorption und Ionisation durch das Massenspektrometer auf eine Massenspektrometersonde aufgebracht wird.

36. Verfahren nach Anspruch 33, wobei das Adsorptionsmittel ausgewählt ist aus einem hydrophilen Adsorptionsmittel und einem Metallchelat-Adsorptionsmittel.

37. Verfahren nach Anspruch 33, wobei das Adsorptionsmittel ein starkes Anionenaustauschadsorptionsmittel oder ein Nickelchelat-Adsorptionsmittel ist.

38. Kit zur Unterstützung bei der Differentialdiagnose von Prostatakrebs, benigner Prostatahyperplasie und einer negativen Diagnose, umfassend:
(1) ein an ein Substrat gebundenes Adsorptionsmittel, wobei das Adsorptionsmittel wenigstens einen Proteinmarker zurückhält, der ausgewählt ist aus:
Marker SP1: 9.402,68 ± 8,97 Da und
Marker SP3: 54.979,27 ± 408,78 Da; und
(2) Instruktionen zum Nachweisen des Markers oder der Marker durch In-Kontakt-Bringen einer Probe mit dem Adsorptionsmittel und Nachweisen des von dem Adsorptionsmittel zurückgehaltenen Markers oder der von dem Adsorptionsmittel zurückgehaltenen Marker; wobei das Adsorptionsmittel wenigstens einen Antikörper umfasst, der ausgewählt ist aus einem Antikörper, der spezifisch Marker SP1 bindet, und einem Antikörper, der spezifisch Marker SP3 bindet.

39. Kit nach Anspruch 38, wobei das Adsorptionsmittel wenigstens einen weiteren Proteinmarker zurückhält, der ausgewählt ist aus:
Marker SP2: 26.155,30 ± 202,01 Da,
Marker SP5: 8.766,93 ± 14,44 Da,
Marker SP6: 6.277,97 ± 12,36 Da und
Marker SP7: 2.781,72 ± 4,41 Da.

40. Kit nach Anspruch 38 oder 39, wobei das Adsorptionsmittel wenigstens einen weiteren Proteinmarker zurückhält, der ausgewählt ist aus:
Marker CL1: 8.494,30 ± 10,24 Da und
Marker CL2: 9.614,62 ± 52,19 Da.

41. Kit nach irgendeinem der Ansprüche 38-40, wobei das Adsorptionsmittel wenigstens einen weiteren Proteinmarker zurückhält, der ausgewählt ist aus:
Marker CL3: 28.472 ± 127,40 Da und
Marker CL4: 33.386,85 ± 160,47 Da.

42. Kit nach irgendeinem der Ansprüche 38-41, wobei das Substrat eine Sonde für ein Gasphasenionenspektrometer ist, die eine Oberfläche hat, an die das Adsorptionsmittel gebunden ist.

43. Kit nach irgendeinem der Ansprüche 38-41, ferner umfassend:
(i) ein Elutionsmittel, wobei der Marker oder die Marker beim Waschen mit dem Elutionsmittel an dem Adsorptionsmittel zurückgehalten werden; oder
(ii) Instruktionen zum Waschen des Adsorptionsmittels mit dem Elutionsmittel nach In-Kontakt-Bringen des Adsorptionsmittels mit dem Marker oder den Markern.

44. Kit nach irgendeinem der Ansprüche 38-43, wobei das Adsorptionsmittel ferner ein hydrophiles Adsorptionsmittel oder ein Metallchelat-Adsorptionsmittel umfasst.

45. Kit nach Anspruch 44, wobei das hydrophile Adsorptionsmittel ferner Siliciumoxid umfasst.

46. Kit nach irgendeinem der Ansprüche 38-43, wobei das Adsorptionsmittel ferner ein Anionenaustauschadsorptionsmittel umfasst.

47. Kit nach irgendeinem der Ansprüche 38-43, wobei das Adsorptionsmittel ferner ein Nickelmetallchelat-Adsorptionsmittel umfasst.

## Revendications

1. Procédé d'aide dans un diagnostic différentiel du cancer de la prostate, de l'hyperplasie prostatique bénigne, et d'un diagnostic négatif, comprenant :
(a) la détection d'au moins un marqueur protéique dans un premier échantillon provenant d'un sujet, le marqueur protéique étant choisi parmi :
le marqueur SP1 : 9 402,68 ± 8,97 Da, et
le marqueur SP3 : 54 979,27 ± 408,78 Da ; et
(b) la corrélation de la détection du ou des marqueur(s) de l'étape (a) avec un diagnostic probable de cancer de la prostate, d'hyperplasie prostatique bénigne ou un diagnostic négatif, la corrélation prenant en compte la capacité de détection relative du ou des marqueur(s) dans chaque diagnostic.

2. Procédé selon la revendication 1, comprenant en outre la détection d'au moins un marqueur protéique dans ledit premier échantillon choisi parmi :
le marqueur SP2 : 26 155,30 ± 202,01 Da,
le marqueur SP5 : 8 766,93 ± 14,44 Da,
le marqueur SP6 : 6 277,97 ± 12,36 Da, et
le marqueur SP7 : 2 781,72 ± 4,41 Da.

3. Procédé selon la revendication 1 ou la revendication 2, comprenant en outre la détection d'au moins un marqueur protéique dans un deuxième échantillon dudit sujet, ledit marqueur protéique étant choisi parmi :
le marqueur CL1 : 8 494,30 ± 10,24 Da, et
le marqueur CL2 : 9 614,62 ± 52,19 Da.

4. Procédé selon l'une quelconque des revendications 1 à 3, comprenant en outre la mesure de la quantité de marqueur CL3 : 28 472 ± 127,40 Da et de marqueur CL4 : 33 386,85 ± 160,47 Da dans un deuxième échantillon dudit sujet, et la détermination du rapport des quantités de marqueur CL4 au marqueur CL3, et la corrélation du rapport avec un diagnostic probable de cancer de la prostate, d'hyperplasie prostatique bénigne ou un diagnostic négatif, la corrélation prenant en compte la capacité de détection relative du ou des marqueur(s) dans chaque diagnostic.

5. Procédé selon l'une quelconque des revendications précédentes, comprenant la détection du ou des marqueur(s) par spectrométrie ionique en phase gazeuse.

6. Procédé selon la revendication 5, dans lequel ladite spectrométrie ionique en phase gazeuse est la spectrométrie de masse à désorption laser.

7. Procédé selon l'une quelconque des revendications 1 à 4, comprenant la détection du ou des marqueur(s) par dosage immunologique.

8. Procédé selon l'une quelconque des revendications 1 à 4, dans lequel ledit premier échantillon est le plasma séminal.

9. Procédé selon l'une quelconque des revendications 1 à 4, dans lequel ledit premier échantillon est choisi dans le groupe comprenant le sang, le sérum, l'urine, le fluide prostatique, le fluide séminal, le sperme et le tissu prostatique.

10. Procédé selon l'une quelconque des revendications précédentes, comprenant la détection du marqueur SP2 et du marqueur SP1.

11. Procédé selon l'une quelconque des revendications 1 à 9, comprenant la détection du marqueur SP2.

12. Procédé selon la revendication 11, comprenant en outre la détection d'au moins l'un ou de deux marqueurs parmi SP1 et un marqueur choisi parmi : 9 752,30 ± 15,08 Da, SP5, SP6 et SP7.

13. Procédé selon la revendication 12, comprenant la détection du marqueur SP2, du marqueur SP1 et d'un marqueur choisi dans le groupe consistant en SP4, SP5, SP6 et SP7.

14. Procédé selon la revendication 13, comprenant la détection du marqueur SP4.

15. Procédé selon la revendication 13, comprenant la détection du marqueur SP5.

16. Procédé selon la revendication 13, comprenant la détection du marqueur SP6.

17. Procédé selon la revendication 13, comprenant la détection du marqueur SP7.

18. Procédé selon l'une quelconque des revendications 3 à 17, dans lequel ledit deuxième échantillon est un extrait de tissu prostatique.

19. Procédé selon l'une quelconque des revendications 3 à 17, dans lequel ledit deuxième échantillon est choisi dans le groupe comprenant le sang, le sérum, l'urine, le fluide prostatique, le fluide séminal, le sperme, le plasma séminal et le tissu prostatique.

20. Procédé selon l'une quelconque des revendications précédentes, comprenant la détection du marqueur CL2 : 9 614,62 ± 52,19 Da.

21. Procédé selon l'une quelconque des revendications précédentes, comprenant la détection du marqueur CL1 : 8 494,30 ± 10,24 Da.

22. Procédé selon l'une quelconque des revendications précédentes, comprenant la détection du marqueur CL2 : 9 614,62 ± 52,19 Da et du marqueur CL1 : 8 494,30 ± 10,24 Da.

23. Procédé selon la revendication 6, comprenant :
(i) la génération de données sur l'échantillon avec le spectromètre de masse indiquant l'intensité du signal du rapport masse/charge,
(ii) la transformation des données sous une forme informatique,
(iii) l'exécution d'un algorithme avec un ordinateur numérique programmable, dans lequel l'algorithme détermine la qualité d'ajustement entre les données informatiques et les données indiquant un diagnostic de CaP, BPH ou un diagnostic négatif.

24. Procédé selon la revendication 6, comprenant :
(i) la génération de données sur l'échantillon avec le spectromètre de masse indiquant l'intensité du signal du rapport masse/charge,
(ii) la transformation des données sous une forme informatique ; et
(iii) l'exécution d'un algorithme avec un ordinateur numérique programmable, dans lequel l'algorithme détecte un signal dans les données informatiques représentant le ou les marqueur(s).

25. Procédé selon la revendication 6, dans lequel la spectrométrie de masse par désorption laser/ionisation comprend :
(i) la fourniture d'une sonde adaptée pour l'utilisation avec un spectromètre de masse comprenant un adsorbant fixé sur celle-ci ;
(ii) le contact du ou des marqueur(s) avec l'adsorbant ; et
(iii) la désorption et l'ionisation du ou des marqueur(s) de la sonde et la détection du ou des marqueur(s) désorbé(s)/ionisé(s) avec le spectromètre de masse.

26. Procédé selon la revendication 6, dans lequel la spectrométrie de masse par désorption laser/ionisation comprend :
(i) la fourniture d'un substrat comprenant un adsorbant fixé sur celui-ci ;
(ii) le contact du ou des marqueur(s) avec l'adsorbant ;
(iii) le placement du substrat sur une sonde adaptée pour l'utilisation avec un spectromètre de masse comprenant un adsorbant fixé sur celle-ci ; et
(iv) la désorption et l'ionisation du ou des marqueur(s) de la sonde et la détection du ou des marqueur(s) désorbé(s)/ionisé(s) avec le spectromètre de masse.

27. Procédé selon la revendication 25, dans lequel l'adsorbant est un adsorbant hydrophile ou un adsorbant chélateur métallique.

28. Procédé selon la revendication 27, dans lequel l'adsorbant est un adsorbant hydrophile comprenant l'oxyde de silicium.

29. Procédé selon la revendication 27, dans lequel l'adsorbant est un adsorbant chélateur métallique comprenant du nickel.

30. Procédé selon la revendication 25, dans lequel l'adsorbant comprend un anticorps qui se lie spécifiquement au marqueur.

31. Procédé selon la revendication 5, comprenant en outre, avant la détection du ou des marqueur(s), le fractionnement de l'échantillon par chromatographie par perméation de gel et le recueil d'une fraction comprenant le ou les marqueur(s).

32. Procédé selon la revendication 5, le procédé comprenant en outre, avant la détection du ou des marqueur(s), le fractionnement de l'échantillon par chromatographie par échange d'anions et le recueil d'une fraction comprenant le ou les marqueur(s).

33. Procédé selon la revendication 5, dans lequel, si les marqueurs CL1, CL2, CL3 ou CL4 sont détectés, le procédé comprend en outre,
(i) avant la détection du ou des marqueur(s), le fractionnement d'un échantillon comprenant le ou les marqueur(s) en mettant en contact l'échantillon avec un substrat comprenant un adsorbant qui retient le ou les marqueur(s) et en éliminant l'échantillon non retenu ; et
(ii) la désorption et l'ionisation des marqueurs retenus de l'adsorbant pendant la spectrométrie de masse.

34. Procédé selon la revendication 33, dans lequel le substrat est une sonde de spectromètre de masse comprenant l'adsorbant sur une surface de sonde.

35. Procédé selon la revendication 34, dans lequel le substrat est une résine et, après fractionnement de l'échantillon, la résine avec le ou les marqueur(s) retenu(s) par l'adsorbant est placée sur une sonde de spectromètre de masse pour désorption et ionisation par le spectromètre de masse.

36. Procédé selon la revendication 33, dans lequel l'adsorbant est choisi parmi un adsorbant hydrophile et un adsorbant chélateur métallique.

37. Procédé selon la revendication 33, dans lequel l'adsorbant est un adsorbant par échange d'anions puissant ou un adsorbant chélateur à base de nickel.

38. Kit pour aider au diagnostic différentiel du cancer de la prostate, de l'hyperplasie prostatique bénigne et d'un diagnostic négatif, comprenant :
(1) un adsorbant fixé à un substrat, l'adsorbant retenant au moins un marqueur protéique choisi parmi :
le marqueur SP1 : 9 402,68 ± 8,97 Da, et
le marqueur SP3 : 54 979,27 ± 408,78 Da ; et
(2) des instructions pour détecter le ou les marqueur(s) en mettant en contact un échantillon avec l'adsorbant et en détectant le ou les marqueur(s) retenu(s) par l'adsorbant, ledit adsorbant comprenant au moins un anticorps choisi parmi un anticorps qui se lie spécifiquement au marqueur SP1 et un anticorps qui se lie spécifiquement au marqueur SP3.

39. Kit selon la revendication 38, dans laquelle ledit adsorbant retient au moins un autre marqueur protéique choisi parmi :
le marqueur SP2 : 26 155,30 ± 202,01 Da,
le marqueur SP5 : 8 766,93 ± 14,44 Da,
le marqueur SP6 : 6 277,97 ± 12,36 Da, et
le marqueur SP7 : 2 781,72 ± 4,41 Da.

40. Kit selon la revendication 38 ou 39, dans laquelle ledit adsorbant retient au moins un autre marqueur protéique choisi parmi :
le marqueur CL1 : 8 494,30 ± 10,24 Da, et
le marqueur CL2 : 9 614,62 ± 52,19 Da.

41. Kit selon l'une quelconque des revendications 38 à 40, dans laquelle ledit adsorbant retient au moins un autre marqueur protéique choisi parmi :
le marqueur CL3 : 28 472 ± 127,40 Da, et
le marqueur CL4 : 33 386,85 ± 160,47 Da.

42. Kit selon l'une quelconque des revendications 38 à 41, dans laquelle le substrat est une sonde pour spectromètre ionique en phase gazeuse présentant une surface sur laquelle l'adsorbant est fixé.

43. Kit selon l'une quelconque des revendications 38 à 41, comprenant en outre
(i) un éluant dans lequel le ou les marqueur(s) sont retenus sur l'adsorbant lorsqu'ils sont lavés avec l'éluant, ou
(ii) des instructions pour laver l'adsorbant avec l'éluant après le contact de l'adsorbant avec le ou les marqueur(s).

44. Kit selon l'une quelconque des revendications 38 à 43, dans laquelle l'adsorbant comprend en outre un adsorbant hydrophile ou un adsorbant chélateur métallique.

45. Kit selon la revendication 44, dans laquelle l'adsorbant hydrophile comprend en outre de l'oxyde de silicium.

46. Kit selon l'une quelconque des revendications 38 à 43, dans laquelle l'adsorbant comprend en outre un adsorbant par échange d'anions.

47. Kit selon l'une quelconque des revendications 38 à 43, dans laquelle l'adsorbant comprend en outre un adsorbant chélateur métallique à base de nickel.
